(19)

**Europäisches Patentamt**
**European Patent Office**
**Office européen des brevets**

(11)  **EP 1 850 859 B1**

(12)  **EUROPEAN PATENT SPECIFICATION**

(45) Date of publication and mention
of the grant of the patent:
**20.07.2016  Bulletin 2016/29**

(51) Int Cl.:
*A61K 33/24* (2006.01)          *A61K 33/26* (2006.01)
*A61P 13/00* (2006.01)

(21) Application number: **06709690.9**

(22) Date of filing: **09.02.2006**

(86) International application number:
**PCT/GB2006/000452**

(87) International publication number:
**WO 2006/085079 (17.08.2006 Gazette 2006/33)**

(54) **PHARMACEUTICALLY ACTIVE PHOSPHATE BINDERS, THEIR MANUFACTURE, COMPOSITIONS CONTAINING THEM AND THEIR USE**

PHARMAZEUTISCH WIRKSAME PHOSPHATBINDER, IHRE HERSTELLUNG, DIESE ENTHALTENDE ZUSAMMENSETZUNGEN UND IHRE VERWENDUNG

COMPOSES PHARMACEUTIQUEMENT ACTIFS, LEUR FABRICATION, COMPOSITIONS CONTENANT CES COMPOSES ET LEUR UTILISATION

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **10.02.2005  GB 0502787**

(43) Date of publication of application:
**07.11.2007  Bulletin 2007/45**

(60) Divisional application:
**16174070.9**

(73) Proprietor: **OPKO Ireland Global Holdings, Limited Dublin 24 (IE)**

(72) Inventors:
• **NEWTON, Maurice, Sydney**
  **Sandbach Cheshire CW11 3LU (GB)**
• **RHODES, Nigel Peter**
  **Grappenhall,**
  **Warrington Cheshire WA4 2QS (GB)**

• **TOFT, Alexis John**
  **Thelwall,**
  **Warrington Cheshire WA4 2SZ (GB)**

(74) Representative: **D Young & Co LLP**
  **120 Holborn**
  **London EC1N 2DY (GB)**

(56) References cited:
**WO-A-99/15189          WO-A-2004/016553**
**WO-A-2005/027876      US-A- 4 629 626**

• **TSUGIO SATO, TAKAYOSHI WAKABAYASHI, MASAHIKO SHIMADA: "Adsorption of various anions by magnesium aluminum oxide" IND.ENG.CHEM.PROD.RES.DEV., 1986, pages 89-92, XP002392181**
• **PATENT ABSTRACTS OF JAPAN vol. 2003, no. 12, 5 December 2003 (2003-12-05) & JP 2004 089760 A (NATIONAL INSTITUTE OF ADVANCED INDUSTRIAL & TECHNOLOGY), 25 March 2004 (2004-03-25) cited in the application**

**Description**

FIELD OF THE INVENTION

The present invention relates to mixed metal compounds having pharmaceutical activity, especially as phosphate binders. It also extends to methods of manufacture of those compounds, as well as to pharmaceutical compositions containing such compounds. It further relates to their pharmaceutical use.

BACKGROUND OF THE INVENTION

In patients with kidney failure on haemodialysis, phosphate concentrations in the blood plasma can rise dramatically and this condition, known as hyperphosphataemia, can result in calcium phosphate deposition in soft tissue. Plasma phosphate levels may be reduced by oral intake of inorganic and organic phosphate binders. One of the most common treatments involves dosing with aluminium hydroxide gel which forms an insoluble aluminium phosphate. However, this can result in further toxic complications due to Aluminium accumulation, e.g. reduction in haemoglobin production, impairment in natural repair and production of bone and possible impairment of neurological/cognitive function. Other aluminium compounds such as microcrystalline aluminium oxide hydroxide (boehmite) and certain hydrotalcites have been proposed for this use, such as disclosed in Ookubo et al, Journal Pharmaceutical Sciences (November 1992), 81(11), 1139-1140. However, these suffer from the same drawbacks.

Many known inorganic preparations for treatment of hyperphosphataemia are efficient phosphate binders only over a limited pH range, especially an acid pH range of about 3-5. Such phosphate binders effective at pH 3 would not necessarily bind as effectively at higher pH, e.g. $\geq$ 7, which are found in the lower digestive tract, e.g. duodenum and below, and where at least some of the binding of phosphate may take place. Moreover, particularly alkaline binders could buffer the stomach pH up to a high level at which they would not have a phosphate binding capacity.

To overcome the drawbacks associated with aluminium and also problems of efficacy over a limited pH range, WO-A-99/15189 discloses use of mixed metal compounds which are free from aluminium and which have a phosphate binding capacity of at least 30% by weight of the total weight of phosphate present, over a pH range of from 2-8.

Typically, such mixed metal compounds may contain iron (III) and at least one of magnesium, calcium, lanthanum and cerium. Preferably they also contain at least one of hydroxyl and carbonate anions and optionally additionally, at least one of sulphate, nitrate, chloride and oxide. However, we have found that the mixed metal compounds of WO-A-99/15189 release some of their divalent metal content in soluble form during use.

JP-A-2004-89760 discloses enhancement of the dephosporising activity of certain mixed metal compounds for removal of phosphorus from domestic or industrial waste water by heat treatment of crystals of such compounds which are defined as having the general formula:

$$M^{II}_{1-x}M^{III}_{x}(OH)_2 A^{n-}_{y}.mH_2O$$

where $M^{II}$ is at least one bivalent metal; $M^{III}$ is at least one trivalent metal; $A^{n-}$ is a n-valent anion; and x, y and m fulfil $0 < x \leq 0.67, 0 < y \leq 1, 0 \leq m \leq 2$. Such compounds are said to have a coefficient of selection for "sulphate ions of phosphorus" dissolved in water of at least 5.

A preferred method of preparing such heat treated compounds entails using a mixed aqueous solution of a water-soluble salt of an inorganic or organic acid and alkali hydroxide which is added drop wise to an aqueous solution containing a water-soluble compound of bivalent metal and a water-soluble compound of trivalent metal or of bivalent manganese and reacted at a temperature kept at 0~90°C to obtain crystals of the compound metal hydroxide expressed by the aforementioned general formula by precipitation. This precipitate is separated-off and heat-treated at 200-500°C.

Loss of (trivalent) aluminium from MgAl LDH during phosphate desorption, as well as heat treatment of MgMn LDH compounds is disclosed in Tezuka, S., Bull. Chem. Soc. Jpn., 77 (2004). 2101-7.

We have now found that the release of the divalent metal, e.g. magnesium, associated with the pharmaceutical use of compounds of WO-A-99/15189 can be significantly reduced by heat treatment of a suitable mixed metal compound, for example a layered double hydroxide or a compound having a hydrotalcite structure. It can similarly reduce the release of other bivalent metals when $M^{II}$ is other than magnesium.

By the term "mixed metal compound" is meant a single substance containing two or more different metal types. A single substance generally cannot be separated into its component elements by physical separation methods but requires a chemical reaction.

As used herein, the term "Layered Double Hydroxide" (LDH) is used to designate synthetic or natural lamellar hydroxides with two kinds of metallic cations in the main layers and interlayer domains containing anionic species. This wide family of compounds is sometime also referred to as anionic clays, by comparison with the more usual cationic clays whose interlamellar domains contain cationic species. LDHs have also been reported as hydrotalcite-like compounds by reference to one of the polytypes of the corresponding [Mg-Al] based mineral. (See "Layered Double Hydroxides: Present and Future", ed, V Rives, 2001 pub. Nova Science)

## DEFINITION OF THE INVENTION

A first aspect of the present invention now provides a substance for use as a medicament as defined in claim 1.

In the above formula (I), when A represents more than one anion, the valency (i.e. the charge of the anion) (n) of each may vary.

In the above formula (I), "$\Sigma cn$" means the sum of the number of moles of each anion, per mole of compound of formula (I), multiplied by its respective valency.

The value of z is suitably 2 or less, more preferably 1.8 or less, even more preferably 1.5 or less. The value of z may be 1 or less.

The value of a is suitably from 0.1 to 0.5, preferably from 0.2 to 0.4.

The value of b is suitably 1.5 or less, preferably 1.2 or less. The value of b is preferably greater than 0.2, more preferably greater than 0.4, even more preferably greater than 0.6, most preferably greater than 0,9,

When a is $\geq$ 0.3 it is preferred that $\Sigma cn$ < 0.5a. When a is $\leq$ 0.3 it is preferred that $\Sigma cn$ < 0.7a .

The value of c for each anion is determined by the need for charge neutrality as expressed by the formula $2+a = 2b+\Sigma cn$.

The substance according to the first aspect of the invention preferably comprises greater than 30%, more preferably greater than 50% by weight of a compound or compounds of formula (I), e.g. up to 95% or 90% by weight of the substance.

The process for preparing compounds of formula (I) results in changes in the structural detail of the compound which is the starting material. Therefore the formula (I) as written is only intended to describe its elemental composition and should not be taken as a definition of structure.

When the compound of formula (I) comprises magnesium as $M^{II}$ and iron as $M^{III}$ cations and carbonate as an anion, preferably it exhibits an x-ray diffraction peak at 34° $2\theta$. At lower temperatures ($\leq$ 250 °C), conflicting peaks from the layered double hydroxide may be present whereas when the temperature rises ($\geq$400 °C) , a conflicting peak due to the oxide $M^{II}O$ may appear but these peaks may be resolved using deconvolution methods.

These preferred values for the substance and compound of the first aspect of the invention apply to the other aspects of the invention as described herein. A second aspect of the present invention provides a substance for use as a medicament as defined in claim 7. Preferably, the starting material comprises a compound of formula (II):

$$M^{II}_{1-x}M^{III}_{x}(OH)_2A^{n-}_{y}.mH_20 \qquad (II)$$

where $M^{II}$ is at least one bivalent metal (i.e. with 2 positive charges); $M^{III}$ is at least one trivalent metal (i.e. with three positive charges); An- is at least one n-valent anion; x= $\Sigma ny$; and x and m fulfil $0 < x \leq 0.5$, $0 \leq m \leq 10$.

It should be noted that formula (II) is to be interpreted in such a way as to preserve overall charge neutrality. In formula (I) and/or formula (II) sub- classes of compounds of either formula may comprise, respectively, those wherein a or x is less than any of the following values and those wherein a or x is greater than or equal to any of those values, these values being 0.1, 0.15, 0.2, 0.25, 0.3, 0.35, 0.4, 0.45. One such example comprises the subclasses, wherein a is, respectively, greater than or equal to 0.3, and less than 0.3.The value of x is suitably from 0.1 to 0.5, preferably from 0.2 to 0.4.

In formula (II), $\Sigma ny$ is the sum of the number of each anion multiplied by its respective valency.

The heating at a temperature of 250°C to 500°C of a compound of formula (II) preferably results in a reduction in the amount of metal $M^{II}$ lost to solution by at least 50% by weight compared to that lost from the unheated compound of formula (II), under the conditions described in more detail herein. This preference applies to any aspect of the invention involving formula (II).

The heating is suitably carried out in a heated environment at 250°C to 500°C for a period of 1 minute or longer, more preferably 5 minutes or longer, more preferably 1 hour or longer. The compound is in the heated environment for 3 hours or less.

The heating as described above results in the calcination of the compound according to formula (II). The calcination is believed to lead to the formation of a substance according to the first aspect of the invention. This results in the value of a for a compound according to formula (I) being less than or equal to the value of x for the corresponding untreated compound according to formula (I).

The calcination is preferably not excessive in terms of temperature and/or time of calcination, by which it is meant that the calcination temperature should not exceed 600°C for more than 3 hours, otherwise a phosphate binding performance which is less than optimal may be found.

Excessive calcination results in the reduction of the value of $\Sigma cn/a$ from formula (I) to less than 0.03. Hence it

is preferred that $\Sigma cn/a$ is greater than 0.03, more preferably greater than 0.05, even more preferably greater than 0.09, most preferably greater than 0.10. Excessive calcination also may lead to the formation of a Spinel crystalline structure, hence it is preferred that the substances of the invention do not exhibit a Spinel structure by x-ray diffraction. Spinel has a value for a of 0.67 and so it is preferred if the compound of formula (I) has a value for a of 0.66 or less, preferably 0.5 or less, more preferably 0.5 or less.

Preferably calcination of the compound of formula (II) results in a substance with at least a 10% higher phosphate binding capacity relative to that of the compound of formula (II) from which the substance is obtained or obtainable by calcination. The preferred values describe hereinabove also apply to the other aspect of the invention described below.

A suitable method for monitoring the degree of calcination is by measurement of the percentage loss of crystalline surface water at 105°C. This is measured by allowing a sample to reach an equilibrium moisture content by storage for several days at ambient conditions (20°C, 20% RH), weighing the sample, then heating at 105°C for 4 hours and reweighing to establish the loss in weight, expressed as a percentage. Drying at 105° C removes the surface absorbed water (i.e non-chemically-bound water or water on the crystal surface)

Suitably, the mixed metal compound after calcination has less than 2%, preferably less than 1.5%, more preferably less than 1% by weight crystallite-surface absorbed water.

For convenience, any substance for use as a medicament as hereinbefore defined in accordance with the first or the second aspect of the present invention is hereinafter referred to as a "substance of the invention".

These preferred values for the substance and compound of the first and second aspects of the invention apply to the other aspects of the invention as described herein.

A third aspect of the present invention provides use as defined in claim 12 of a substance comprising a phosphate binding solid mixed metal compound of formula (I):

$$M^{II}_{1-a}M^{III}_aO_bA^{n-}_c.zH_2O \qquad (I)$$

where $M^{II}$ is at least one bivalent metal; $M^{III}$ is $Fe^{3+}$; $A^{n-}$ is at least one n-valent anion; $2+a = 2b+ \Sigma cn$; $a= M'''/(M''+M''')$; and $\Sigma cn<0.9a$ wherein the compound of formula (I) is obtainable by heating a starting material comprising a layered double hydroxide structure at a temperature in a range of 250°C to 500°C for 3 hours or less.

The medicament may be used on animals, preferably humans.

A fourth aspect of the present invention provides a use as defined in claim 13. A fifth aspect of the present invention provides use as defined in claim 16. Preferably, the starting material comprises of a compound of formula (II):

$$M^{II}_{1-x}M^{III}_x(OH)_2A^{n-}_y.mH_2O \qquad (II)$$

where $M^{II}$ is at least one bivalent metal; $M^{III}$ is at least one trivalent metal; $A^{n-}$ is at least one n-valent anion; $x= \Sigma ny$; and x and m fulfil $0<x\leq0.5, 0\leq m\leq 10$. The value of x is preferably from 0.1 to 0.5, more preferably from 0.2 to 0.4.

<u>DETAILED DESCRIPTION OF THE INVENTION</u>

<u>Preparation of Active Substances</u>

Substances of the invention are preferably made by heat treatment of a suitable starting material of formula (II) as hereinbefore defined. Optionally other preparation methods may be employed to prepare the substance of invention such as solid state synthesis, solid-solid reactions or highly intensively milling of single or mixed metal oxides or hydroxides using hydrothermal routes or low temperature routes.

The substances of the invention prepared by heat treatment of a suitable starting material of formula (II) as hereinbefore defined may be prepared by providing a first solution of a water soluble compound of metal $M^{II}$ and a water soluble compound of metal $M^{III}$, the anions being chosen so as not to result in precipitation from the first solution. A second solution is also provided, of a water soluble hydroxide (e.g. NaOH) and a water soluble salt of anion $A^{n-}$ (the cation being chosen so as not to precipitate with the hydroxide or the anion with the metal from the hydroxide). The two solutions are then admixed and the mixed metal compound starting material is formed by co-precipitation. It comprises solid crystalline material, usually also with presence of some solid amorphous material. Preferably, at least some of the material so formed is of a layered double hydroxide and/or of a hydrotalcite structure, usually also with some amorphous and/or poorly crystalline material, preferably after co-precipitation, the material is then filtered or centrifuged, washed then dried by heating.

It is preferred that the material is washed in order to remove the water-soluble salts that are the by product of the precipitation reaction. If significant amounts of these soluble salts are left admixed with the solid precipitate, then the subsequent heating of the material may result in the incorporation of the soluble salts into the resulting solid, potentially having an adverse effect on its phosphate binding behaviour. The material is preferably washed such that the remaining level of water soluble salts (having a solubility in water of 1g/litre or more) is less than 15%, preferably less than 10%, more preferably less than 5% by weight of the solid mixed metal compound after drying as described below.

After the filtering or centrifuging and washing, the drying is preferably carried out at low temperature (such as up to 120°C), for example by oven drying, spray drying or fluid bed drying.

Optionally, the dry material may be treated prior to heat treatment, to remove oversize particles by milling and/or sieving and/or any other suitable technique, for example to restrict the material to be heat treated to particles which are substantially no greater than $100\mu m$ in diameter. Preferably, as measured by sieving, less than 10% by weight of particles are greater than $106\mu m$ in diameter, more preferably less than 5%. Most preferably, no particles are greater than $106\mu m$ in diameter as measured by sieving.

The resultant dry material is then directly subjected to the necessary heat treatment, at a temperature of from 250°C to 500°C, for example by means of oven drying or drying in a rotary calcinator or fluid bed dryer.

Optionally, the wet cake material may be directly subjected to temperatures above 200°C without low temperature drying (such as up to 120°C) and milling.

A sixth aspect of the present invention therefore provides a method of preparing a substance for use as a medicament as defined in claim 21. Preferably, the starting material comprises a compound of formula (II):

$$M^{II}_{1-x}M^{III}(OH)_2 A^{n-}_y . mH2O \qquad (II)$$

where $M^{II}$ is at least one bivalent metal; $M^{III}$ is at least one trivalent metal; $A^{n-}$ is at least one n-valent anion; $x=\Sigma ny$; and x and m fulfil $0 < x \leq 0.5$, $0 \leq m \leq 10$. The value of x is preferably from 0.1 to 0.5, more preferably from 0.2 to 0.4.

Preferably, the heating results in a reduction in the amount of loss into solution of metal $M^{II}$ from the heat-treated compound by at least 50% by weight compared to loss from the untreated compound, when measuring the loss of metal $M^{II}$ using the test as hereinafter described.

The substances of the invention may contain at least one compound of formula (I) but the process mentioned above for making the starting material may also cause other materials to be present in the intermediate product e.g. of formula (II) and in the final product, for example single (as opposed to mixed) metal compounds which may also be formed during the co-precipitation process.

The Solid Mixed Metal Compounds

In formula (I) and formula (II), $M^{II}$ is preferably selected from Mg, Zn, Fe (II), Cu (II) and Ni(II). Of these, Mg is especially preferred. $M^{III}$ is preferably selected from Mn(III), Fe(III), La(III) and Ce(III). Of these, Fe(III) is especially preferred, particularly in the case when $M^{II}$ is Mg. $M^{II}$ and $M^{III}$ may be different metals or they may be the same metal but in different valence states. For instance, $M^{II}$ might be Fe(II) and $M^{III}$ Fe(III). However it is highly preferred that $M^{II}$ and $M^{III}$ are different metals. M(III) may also be Al(III) for treatments where aluminium accumulation and toxic complications are not a problem.

$A^{n-}$ preferably comprises at least one anion selected from carbonate, hydrogencarbonate, sulphate, nitrate, halide and hydroxide. Of these, carbonate is especially preferred.

Preferably, any substance of the invention is substantially or totally free of aluminium.

Determination of Phosphate Binding Capacity

A specific method for determining phosphate binding capacity is given in more detail herein. This was the method actually used in the Examples. However, as a generality, elsewhere in this specification, unless specifically indicated to the contrary, any reference to percentage phosphate binding capacity is preferably that determined by the following method. 0.4 gram of the substance of the invention is added to 10ml, 40 mmol $l^{-1}$ sodium phosphate solution adjusted to a pH of choice. Preferably, any quoted percentage phosphate binding capacity herein is maintained for measurements at pH values over the range of from 3 to 7, more preferably from 2 to 8. Samples are homogenised and gently agitated at room temperature (20 °C) for 30 minutes. Following centrifugation for 5 min at 3000 rpm, the supernatant is filtered through $0.22\mu m$ millipore filters. Soluble phosphate is measured in the supernatant. The percentage phosphate bound by the phosphate binder is then calculated relative to the untreated phosphate starting solution.

Formulations

The invention also relates to a pharmaceutical composition which comprises as active ingredient, at least one substance of the invention together with a pharmaceutically acceptable carrier therefor.

Thus, a seventh aspect of the present invention provides a pharmaceutical formulation as defined in claim 26 comprising a substance according to the first aspect of the invention, i.e. a phosphate binding solid mixed metal compound of formula (I):

$$M^{II}_{1-a}M^{II}_aO_bA^{n-}_c.zH_2O \qquad (I)$$

where $M^{II}$ is at least one bivalent metal; $M^{III}$ is $Fe^{3+}$; $A^{n-}$ is at least one n-valent anion; $2+a = 2b+\Sigma cn$; a= number of moles of M'''/(number of moles of M''+number of moles of M'''); and $\Sigma cn< 0.9a$ wherein the compound of formula (I) is obtainable by heating a starting material comprising a layered double hydroxide structure at a temperature in a range of 250°C to 500°C for 3 hours or less..

An eighth aspect of the present invention provides a pharmaceutical formulation as defined in claim 29. Preferably, the starting material comprises a compound of formula (II):

$$M^{II}_{1-x}M^{III}_x(OH)_2A^{n-}_y.mH_2O \qquad (II)$$

where $M^{II}$ is at least one bivalent metal; $M^{III}$ is at least one trivalent metal; $A^{n-}$ is at least one n-valent anion; x=$\Sigma$ny; and x and m fulfil $0 < x \leq 0.5$, $0 \leq m \leq 10$. The value of x is preferably from 0.1 to 0.5, more preferably from 0.2 to 0.4.

A process for the preparation of a pharmaceutical composition as defined above is also provided which comprises bringing at least one substance of the invention into association with a pharmaceutically acceptable carrier and optionally, any other ingredients including by-products resulting from manufacture of the active ingredient.

A pharmaceutically acceptable carrier may be any material with which the substance of the invention is formulated to facilitate its administration. A carrier may be a solid or a liquid, including a material which is normally gaseous but which has been compressed to form a liquid, and any of the carriers normally used in formulating pharmaceutical compositions may be used. Preferably, compositions according to the invention contain 0.5% to 95% by weight of active ingredient. The term pharmaceutically acceptable carrier encompasses diluents, excipients or adjuvants.

Another aspect of the disclosure provides a method for the binding of excess phosphate in animals, particularly humans. In particular it provides a method for the prophylaxis or treatment of hyperphosphataemia in animals, particularly humans. The method comprises the step of administering a substance of the invention, preferably by oral administration.

An aspect of the invention provides the use of a substance of the invention in the preparation of a medicament for the binding of phosphate in animals, preferably humans, in need thereof, preferably for the prophylaxis or treatment of hyperphosphataemia in animals, preferably humans.

The substances of the invention can be formulated in any suitable pharmaceutical composition form but especially in a form suitable for oral administration for example in solid unit dose form such as tablets, capsules, or in liquid form such as liquid suspensions, especially aqueous suspensions. However, dosage forms adapted for extra-corporeal or even intravenous administration are also possible. Suitable formulations can be produced by known methods using conventional solid carriers such as, for example, lactose, starch or talcum or liquid carriers such as, for example, water, fatty oils or liquid paraffins. Other carriers which may be used include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; polypeptide/protein or polysaccharide complexes such as gelatin-acacia complexes; sugars such as mannitol, dextrose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

Auxiliary components such as tablet disintegrants, solubilisers, preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include sodium hydrogencarbonate, citric acid, tartaric acid, hydrochloric acid and maleic acid. Suitable sweeteners include aspartame, acesulfame K and thaumatin. Suitable taste-masking agents include sodium hydrogencarbonate, ion-exchange resins, cyclodextrin inclusion compounds, adsorbates or microencapsulated actives.

For treatment of and prophylaxis of hyperphosphataemia, preferably amounts of from 0.1 to 500mg, more preferably from 1 to 200, mg/kg body weight of substance of the invention as active compound are administered daily to obtain the desired results. Nevertheless, it may be necessary from time to time to depart from the amounts mentioned above, depending on the body weight of the patient, the method of application, the animal species of the patient and its individual reaction to the drug or the kind of formulation or the time or interval in which the drug is applied. In special cases, it may be sufficient to use less than the minimum amount given above, whilst in other cases the maximum dose may have to be exceeded. For a larger dose, it may be advisable to divide the dose into several smaller single doses. Ultimately, the dose will depend upon the discretion of the attendant physician. Administration soon before meals, eg.

within one hour before a meal or taken with food will usually be preferred.

A typical single solid unit dose for human adult administration may comprise from 1 mg to 1 g, preferably from 10 mg to 800 mg of substance of the invention.

A solid unit dose form may also comprise a release rate controlling additive. For example, the substance of the invention may be held within a hydrophobic polymer matrix so that it is gradually leached out of the matrix upon contact with body fluids. Alternatively, the substance of the invention may be held within a hydrophilic matrix which gradually or rapidly dissolves in the presence of body fluid. The tablet may comprise two or more layers having different release properties. The layers may be hydrophilic, hydrophobic or a mixture of hydrophilic and hydrophobic layers. Adjacent layers in a multilayer tablet may be separated by an insoluble barrier layer or hydrophilic separation layer. An insoluble barrier layer may be formed of materials used to form the insoluble casing. A hydrophilic separation layer may be formed from a material more soluble than the other layers of the tablet core so that as the separation layer dissolves the release layers of the tablet core are exposed.

Suitable release rate controlling polymers include polymethacrylates, ethylcellulose, hydroxypropylmethylcellulose, methylcellulose, hydroxyethylcellulose, hydroxypropylcellulose, sodium carboxymethylcellulose, calcium carboxymethylcellulose, acrylic acid polymer, polyethylene glycol, polyethylene oxide, carrageenan, cellulose acetate, zein etc.

Suitable materials which swell on contact with aqueous liquids include polymeric materials include from cross-linked sodium carboxymethylcellulose, cross-linked hydroxypropylcellulose, high molecular weight hydroxypropylcellulose, carboxymethylamide, potassium methacrylatedivinylbenzene copolymer, polymethylmethacrylate, cross-linked polyvinylpyrrolidone and high molecular weight polyvinylalcohols.

Solid unit dose forms comprising a substance of the invention may be packaged together in a container or presented in foil strips, blister packs or the like, eg marked with days of the week against respective doses, for patient guidance.

The disclosure is further described below in the following numbered paragraphs:

1. A compound for use as a medicament, wherein the compound is of formula (I):

$$M^{II}_{1-a}M^{III}_aO_bA^{n-}{}_c.zH_2O \qquad (I)$$

where $M^{II}$ is at least one bivalent metal;

$M^{III}$ is at least one trivalent metal;
$A^{n-}$ is at least one n-valent anion;

$$2+a = 2b+\Sigma cn \text{ and } \Sigma cn < 0.9a.$$

1a. A compound for use as a medicament, wherein the compound is of formula (I):

$$M^{II}_{1-a}M^{III}_aO_bA^{n-}{}_c.zH_2O \qquad (I)$$

where $M^{II}$ is at least one bivalent metal;

$M^{III}$ is at least one trivalent metal;
$A^{n-}$ is at least one n-valent anion;

$$2+a = 2b+\Sigma cn \text{ and } \Sigma cn < 0.9a;$$

z is 2 or less, more preferably 1.8 or less, even more preferably 1.5 or less.

1b. A compound for use as a medicament, wherein the compound is of formula (I):

$$M^{II}_{1-a}M^{III}_aO_bA^{n-}{}_c.zH_2O \qquad (I)$$

where $M^{II}$ is at least one bivalent metal;

$M^{III}$ is at least one trivalent metal;
$A^{n-}$ is at least one n-valent anion;
a is from 0.1 to 0.5, preferably from 0.2 to 0.4.
b is 1.5 or less, preferably 1.2 or less.
the value of c for each anion is determined by the need for charge neutrality as expressed by the formula $2+a = 2b+\Sigma cn$.
z is 2 or less, more preferably 1.8 or less, even more preferably 1.5 or less.

2. A compound according to paragraph 1, 1a or 1 b wherein in formula (I), a is > 0.3.

3. A compound according to paragraph 1, 1a or 1 b wherein in formula (I), a is < 0.3.

4. A compound according to paragraph 2, wherein in formula (I), $0.03a < \Sigma cn < 0.5a$.

5. A compound according to paragraph 3, wherein in formula (I), $03a < \Sigma cn < 0.7a$.

6. A compound according to any preceding paragraph, having less than 2%, preferably less than 1.5%, more preferably less than 1% by weight crystallite-surface absorbed water.

7. A compound for use as a medicament, wherein the compound is obtainable by heating a starting material comprising a layered double hydroxide structure at a temperature of from 200°C to 600°C, preferably from 250°C to 500°C.

7a. A compound for use as a medicament, wherein the compound is obtained by heating a starting material comprising a layered double hydroxide structure at a temperature of from 200°C to 600°C, preferably from 250°C to 500°C.

8. A compound according to paragraph 7 or 7a, wherein the starting material comprises a compound of formula (II):

$$M^{II}_{1-x}M^{III}_{x}(OH)_2 A^{n-}_{y}.mH_2O \qquad (II)$$

where $M^{II}$ is at least one bivalent metal;

$M^{III}$ is at least one trivalent metal;
$A^{n-}$ is at least one n-valent anion;
$0 < x \leq 0.5$,
$0 < y \leq 1$ and
$0 < m < 10$;
and wherein preferably $x = \Sigma yn$

9. A compound according to paragraph 8, wherein in formula (II), x is > 0.3.

10. A compound according to paragraph 8, wherein in formula (II), x is < 0.3.

11. A compound according to any of paragraphs 7 to 10, wherein the substance has a 10% higher phosphate binding capacity relative to the compound of formula (II) from which it is obtainable.

12. Use of a compound in a method of preparing a medicament for binding of phosphate, wherein the compound is of formula (I):

$M^{II}_{1-a}M^{III}_{a}O_b A^{n-}_{c}.zH_2O$ (I)
where $M^{II}$ is at least one bivalent metal;
$M^{III}$ is at least one trivalent metal;
$A^{n-}$ is at least one n-valent anion;

$$2+a = 2b+\Sigma cn \text{ and } \Sigma cn < 0.9a.$$

12a. Use of a compound in a method of preparing a medicament for binding of phosphate, wherein the compound is of formula (I):

$$M^{II}_{1-a}M^{III}_aO_bA^{n-}_c \cdot zH_2O \qquad (I)$$

where $M^{II}$ is at least one bivalent metal;

$M^{III}$ is at least one trivalent metal;
$A^{n-}$ is at least one n-valent anion;

$$2+a = 2b+\Sigma cn \text{ and } \Sigma cn < 0.9a;$$

z is 2 or less, more preferably 1.8 or less, even more preferably 1.5 or less.

12b. Use of a compound in a method of preparing a medicament for binding of phosphate, wherein the compound is of formula (I):

$$M^{II}_{1-a}M^{III}_aO_bA^{n-}_c \cdot zH_2O \qquad (I)$$

where $M^{II}$ is at least one bivalent metal;

$M^{III}$ is at least one trivalent metal;
$A^{n-}$ is at least one n-valent anion;
a is from 0.1 to 0.5, preferably from 0.2 to 0.4.
b is 1.5 or less, preferably 1.2 or less.
the value of c for each anion is determined by the need for charge neutrality as expressed by the formula $2+a = 2b+\Sigma cn$.
z is 2 or less, more preferably 1.8 or less, even more preferably 1.5 or less.

13. Use of a compound in a method of preparing a medicament for prophylaxis or treatment of hyperphosphataemia, wherein the compound is of formula (I):

$$M^{II}_{1-a}M^{III}_aO_bA^{n-}_c \cdot zH_2O \qquad (I)$$

where $M^{II}$ is at least one bivalent metal;

$M^{III}$ is at least one trivalent metal;
$A^{n-}$ is at least one n-valent anion;

$$2+a = 2b+\Sigma cn \text{ and } \Sigma cn < 0.9a.$$

13a. Use of a compound in a method of preparing a medicament for prophylaxis or treatment of hyperphosphataemia, wherein the compound is of formula (I):

$$M^{II}_{1-a}M^{III}_aO_bA^{n-}_c \cdot zH_2O \qquad (I)$$

where $M^{II}$ is at least one bivalent metal;

$M^{III}$ is at least one trivalent metal;
$A^{n-}$ is at least one n-valent anion;

$$2+a = 2b+\Sigma cn \text{ and } \Sigma cn < 0.9a;$$

z is 2 or less, more preferably 1.8 or less, even more preferably 1.5 or less.

13b. Use of a compound in a method of preparing a medicament for prophylaxis or treatment of hyperphosphataemia, wherein the compound is of formula (I):

$$M^{II}_{1-a}M^{III}_{a}O_bA^{n-}_{c}.zH_2O \qquad (I)$$

where $M^{II}$ is at least one bivalent metal;

$M^{III}$ is at least one trivalent metal;
$A^{n-}$ is at least one n-valent anion;
a is from 0.1 to 0.5, preferably from 0.2 to 0.4.
b is 1.5 or less, preferably 1.2 or less.
the value of c for each anion is determined by the need for charge neutrality as expressed by the formula $2+a = 2b+\Sigma cn$.
z is 2 or less, more preferably 1.8 or less, even more preferably 1.5 or less.

14. Use, according to any one of paragraphs 12 to 13b, wherein in formula (I), a is > 0.3 and preferably $0.03a < \Sigma cn < 0.5a$.

15. Use according to any one of paragraphs 12 to 13b, wherein in formula (I), a is < 0.3 and preferably $0.03a < \Sigma cn < 0.7a$..

16. Use of a compound in a method of preparing a medicament for binding of phosphate, wherein the compound is obtainable by heating a starting material comprising a layered double hydroxide structure at a temperature of from 200°C to 600°C, preferably from 250°C to 500°C.

16a. Use of a compound in a method of preparing a medicament for binding of phosphate, wherein the compound is obtained by heating a starting material comprising a layered double hydroxide structure at a temperature of from 200°C to 600°C, preferably from 250°C to 500°C.

17. Use of a compound in a method of preparing a medicament for prophylaxis or treatment of hyperphosphataemia, wherein the compound is obtainable by heating a starting material comprising a layered double hydroxide structure at a temperature of from 200°C to 600°C, preferably from 250°C to 500°C.

17a. Use of a compound in a method of preparing a medicament for prophylaxis or treatment of hyperphosphataemia, wherein the compound is obtained by heating a starting material comprising a layered double hydroxide structure at a temperature of from 200°C to 600°C, preferably from 250°C to 500°C.

18. Use according to paragraph 16, 16a, 17 or 17a wherein the starting material comprises a compound of formula (II):

$$M^{II}_{1-x}M^{III}_{x}(OH)_2A^{n-}_{y}.mH_2O \qquad (II)$$

where $M^{II}$ is at least one bivalent metal;

$M^{III}$ is at least one trivalent metal;
$A^{n-}$ is at least one n-valent anion;
$0 < x \leq 0.5$,
$0 < y \leq 1$ and
$0 < m \leq 10$;
and wherein preferably $x = \Sigma yn$

19. Use, according to paragraph 18, wherein in formula (II), x is > 0.3.

20. Use according to paragraph 18, wherein in formula (II), x is < 0.3.

21. A pharmaceutical composition comprising

(i) a compound of formula (I):

$$M^{II}_{1-a}M^{II}_aO_bA^-_c.zH_2O \qquad (I)$$

where $M^{II}$ is at least one bivalent metal;

$M^{III}$ is at least one trivalent metal;
$A^{n-}$ is at least one n-valent anion;

$$2+a = 2b+\Sigma cn \text{ and } \Sigma cn < 0.9a;$$

z is 2 or less, more preferably 1.8 or less, even more preferably 1.5 or less; and

(ii) a pharmaceutically acceptable carrier, diluent, excipient or adjuvant.

22. A pharmaceutical composition comprising

(i) a compound of formula (I):

$$M^{II}_{1-a}M^{III}_aO_bA^{n-}_c.zH_2O \qquad (I)$$

where $M^{II}$ is at least one bivalent metal;

$M^{III}$ is at least one trivalent metal;
$A^{n-}$ is at least one n-valent anion;
a is from 0.1 to 0.5, preferably from 0.2 to 0.4.
b is 1.5 or less, preferably 1.2 or less.
the value of c for each anion is determined by the need for charge neutrality as expressed by the formula $2+a = 2b+\Sigma cn$.
z is 2 or less, more preferably 1.8 or less, even more preferably 1.5 or less; and

(ii) a pharmaceutically acceptable carrier, diluent, excipient or adjuvant.

23. A pharmaceutical composition according to paragraph 21 or 22, wherein in formula (I), a is > 0.3 and preferably $0.03a < \Sigma cn < 0.5a$.

24. A pharmaceutical composition according to paragraph 21 or 22, wherein in formula (I), a is < 0.3 and preferably $0.03a < \Sigma cn < 0.7a$.

25. A pharmaceutical composition comprising

(i) a compound obtainable by heating a starting material comprising a layered double hydroxide structure at a temperature of from 200°C to 600°C, preferably from 250°C to 500°C; and
(ii) a pharmaceutically acceptable carrier, diluent, excipient or adjuvant.

26. A pharmaceutical composition comprising

(i) a compound obtained by heating a starting material comprising a layered double hydroxide structure at a temperature of from 200°C to 600°C, preferably from 250°C to 500°C; and
(ii) a pharmaceutically acceptable carrier, diluent, excipient or adjuvant.

27. A pharmaceutical composition according to paragraph 26, wherein the starting material comprises a compound of formula (II):

$$M^{II}_{1-x}M^{III}_x(OH)_2A^{n-}_y.mH_2O \qquad (II)$$

where $M^{II}$ is at least one bivalent metal;

$M^{III}$ is at least one trivalent metal;
$A^{n-}$ is at least one n-valent anion;
$0 < x \leq 0.5$,
$0 < y \leq 1$ and
$0 < m \leq 10$;
and wherein preferably $x = \Sigma yn$

28. A pharmaceutical composition according to paragraph 27, wherein in formula (II), x is > 0.3.

29. A pharmaceutical composition according to paragraph 27, wherein in formula (II), x is < 0.3.

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments.

The present invention will now be explained in more detail by way of the following non-limiting examples.

EXAMPLES

**Method 1(M1)**

(a) Preparation of Starting Materials

Two starting materials, designated Batch A and Batch B were prepared by the method set out below.

Sodium carbonate (21 kg solid) and sodium hydroxide (25 kg solid) were dissolved in 158 kg de-ionised water(DIW). In a separate vessel 129 kg deionised water 52 kg Magnesium sulphate ($MgSO_4.7H_2O$; solid) and 50 kg iron sulphate solution (supplied by Kemira), 43 % w/w were dissolved to prepare batch A (2:1 ratio Mg:Fe) or alternatively 143 kg de-ionised water was used to dissolve 62 kg Magnesium sulphate and 30 kg iron sulphate solution to prepare batch B (4:1 ratio Mg:Fe). The solutions were then added simultaneously over 60 minutes to a stirred 68 kg cold water pool at controlled flow rates sufficient to maintain pH 10.3 in the reaction mixture, (+/- 0.2pH units), at a reaction temperature not exceeding 30°C. When the additions were complete, the reaction mixture was mixed for another 30 minutes and then filtered using a filter press (plate and frame press). The filtered product was then washed 3 times (39 litres/wash) with cold deionised water(DIW) and blown with compressed air to dewater the solid. After isolation, the product was dried using a large tray-oven at 100-120°C.

The solutions were prepared in clean 200 litre plastic drums using a high-shear mixer to dissolve the solids in DIW.

For the caustic soda solution, the mixing head was completely covered by the water before switching on the mixer. The pearl sodium hydroxide was then added in one lot, followed by the sodium carbonate. The solution was stirred until the entire solid had dissolved.

For the Magnesium/Ferric sulphate solution, the Unishear mixing head was completely covered by the water before switching on the mixer. The magnesium sulphate was added to the water followed by addition of the ferric sulphate solution. The solution was stirred until all of the magnesium sulphate had dissolved.

For precipitation, sufficient DIW (deionised water) was used to just cover the agitator blades in the reactor vessel (this was ca. 68kg of water).

Flow control units were used to deliver the appropriate flow rates of the alkaline carbonate and metal sulphate solution

After starting the propeller-blade mixer in the 100 gallon (ca. 455 litres) reaction-vessel , the feed pumps were started. The alkaline carbonate and metal sulphate solutions then started flowing into the reaction-vessel. During the precipitation the pH of the reaction mixture was monitored using a pH-meter. The target pH was 10.3, (+/- 0.2pH units). It took ca. 5-10 minutes before the pH stabilised within the desired band. The pH was checked at regular intervals during the one-hour addition time, and the flow rates adjusted of either the carbonate or sulphate stream as necessary to alter and control the pH. The temperature was also checked at regular intervals. The reaction temperature increased slowly partly due to the heat of reaction and also because the feed solutions were warm due to the action of the high shear mixer during dissolution or heat of reaction. When the temperature approached 30°C, the cooling water was switched on. The total addition time was 60 minutes. The batch was then stirred for 30 minutes at 25-30°C.

The batch was then filtered on a filter-press. The press batch was then blown with compressed air for ca. 30-60 minutes and then discharged. The cake was then dried at 100-120°C for 16 hours in the tray oven.

Small product samples, for analysis of samples prior to calcination, were ground by using a pestle and mortar. Larger quantities, for samples to be calcined, were ground in a Waring commercial blender (blender 800 E) for not more than 8 minutes.

Samples were classified using a 200mm diameter, stainless steel, certified $106\mu$m sieve/lid/receiver. The sample was sieved by hand, oversize material is returned to the stock dried sample to be reground. Continue until the entire sample is <$106\mu$m in size. Transfer the ground product to a self-seal plastic bag and agitate the sample to thoroughly mix it.

The materials of Batch A and Batch B were targeted to have Mg:Fe ratios of 2:1 and 4:1 respectively, and the actual molecular formulae found by analysis were:

Batch A: $[Mg_{0.67}Fe_{0.33}(OH)_2][(CO_3)_{0.17}(SO_4)_{0.01}.0.43H_2O][Na_2SO_4]_{0.03}$
Batch B: $[Mg_{0.80}Fe_{0.20}(OH)_2][(CO_3)_{0.16}(SO_4)_{0.01}.0.60H_2O][Na_2SO_4]_{0.03}$

(b) Preparation of Example substances according to the Invention

Samples were calcined (heat-treatment of samples up to 1000 °C) by different methods namely; rotary calcination, fluid-bed calcination, and standard static oven calcination. Between 10g and 100 g of ground and sieved samples, according to method 4a and 4b, were placed in the oven. The 2:1 and 4:1 samples tested by the different calcination methods at 500 °C and 3 hours resulted in higher phosphate-binding and lower soluble Mg versus that for the uncalcined material though the improvements varied depending on method and conditions. The fluid bed treatment included an experiment where the sample was over-heated for 15 hours at 500 °C, which resulted in a decrease in phosphate binding versus that for the uncalcined. In contrast, all the samples heat-treated for 3 hours at 500 °C showed an increase in phosphate binding and decrease in soluble magnesium relative to the uncalcined

material. The static oven method was used for calcining samples at different temperatures by placing samples for only 3 hours in the oven.

Mixed metal compound samples weighing between 10-30 gram were placed in a porcelain-type dish (below 500°C) or silica-type dish (for temperatures above 500°C). The diameter of the dishes or bowls varied between 7 and 16 cm. In all experiments the sample-bed was kept below a depth of 1 cm. Four standard type ovens were used, namely; for temperatures up to 500°C: Gallenkamp (Oven300 plus series) and Vectstar (ML016) whereas for temperatures above 500 °C Vectstar (HTL3 and SP14). The samples were left in the pre-heated oven for duration of 3 hours. After 3 hours the calcined samples were placed in a dessicator to cool down. Samples were then stored under dry and cool conditions.

The heat treated materials thus obtained were then tested for phosphate binding capacity, soluble magnesium , surface absorbed water content and also subjected to X-ray diffraction analysis. The methods employed are described below.

Test Method 1: Determination of the Percentage Loss of Crystalline Surface Water at 105 °C.

The samples which had been allowed to equilibriate for several days art 20°C and 20% RH were dried at 105°C for 4 hours and the loss in weight was expressed as a percentage. Drying at 105 °C removes the surface absorbed water (i.e. non-chemically-bound water).

A clean dry weighing dish (crucible) was accurately weighed and the weight recorded (W1). The crucible was filled with from 1.0 to 3.0g of sample and the total weight recorded (W2). The crucible was placed in an oven set at 105 °C for 4 hours. After 4 hours the crucible was removed from the oven, cooled to room temperature in a desiccator, reweighed and the final weight recorded (W3).

$$\text{The \% loss at 105°C} = \frac{(W2 - W3)}{(W2 - W1)} \times 100\%$$

Test Method 2: Determination of Phosphate Binding Capacity and Soluble Magnesium

40mM Sodium Phosphate solution (pH 4) was prepared and treated with the phosphate-binder. The supernatant of the centrifuged phosphate-solution and binder mixture was then diluted and analysed by ICP-OES for Fe, Mg and P content. The latter analysis technique is well known to those skilled in the art. ICP-OES is the acronym for inductively coupled plasma optical emission spectroscopy.

Reagents used for this method were: Sodium Dihydrogen Phosphate Monohydrate (Aldrich), 1M hydrochloric acid, AnalaR™ water, standard phosphorous solution (10.000$\mu$g/ml, Romil Ltd), standard magnesium solution (10,000$\mu$g/ml, Romil Ltd), standard iron solution (1.000$\mu$g/ml), sodium chloride (BDH).

Specific apparatus used were centrifuge (Metler 2000E), blood-tube rotator (Stuart Scientific), minishaker (MS1), ICP-OES, blood collection tubes. Phosphate buffer (pH = 4) was prepared by weighing 5.520 g (+/-0.001 g) of sodium di-hydrogen phosphate followed by addition of AnalaR™ water and transferring to a 1ltr volumetric flask.

To the 1 ltr volumetric flask was then added 1 M HCl drop-wise to adjust the pH to pH 4 (+/-0.1) mixing between additions. The volume was then accurately made up to 1ltr using AnalaR™ water and mixed thoroughly.

0.4g (+/- 0.005g) of each sample was weighed into the supplied blood collection tubes and placed in the holding rack. All samples were prepared in duplicate. 10ml aliquots of the phosphate buffer were pipetted into each of the blood collection tubes containing the pre-weighed test materials and the screw cap fitted. The vessels were agitated over a minishaker for about ten seconds. The vessels were transferred onto a blood tube rotator and mixed for 30 minutes (+/- 2 minutes). The vessels were then centrifuged at 3000rpm and 20°C for 5 minutes. The samples were removed from the centrifuge and 2.5ml aliquots were pipetted of the supernatant and transferred into a fresh blood collection tubes. 7.5 ml of AnalaR™ water were pipetted to each 2.5ml aliquot and the screw cap fitted and mixed thoroughly. The solutions were then analysed on a calibrated ICP-OES.

## The phosphate binding capacity was determined by: phosphate binding (%)

$$=100 - (T_P/S_P \times 100)$$

## Magnesium release was determined by: Magnesium release (mmol/l)

$$=T_{Mg} - S_{Mg}$$

where:

$T_P$ = Analyte value for phosphate in the phosphate solution after reaction with phosphate binder.
$S_P$ = Analyte value for phosphate in the phosphate solution before reaction with phosphate binder.
$T_{Mg}$ = Analyte value for magnesium in the phosphate solution after reaction with phosphate binder.
$S_{Mg}$ = Analyte value for magnesium in the phosphate solution before reaction with phosphate binder.

Test Method 3: X-Ray Diffraction (XRD) measurements

Data was collected for fine particulate samples from 2-70° 2θ on a Philips automatic powder X-ray diffractometer using Copper K alpha radiation generated at 40kV and 55mA.

Experimental Test Results

The results of the above tests performed on the heat treated materials resulting from the starting materials of Batch A and Batch B are presented below in Table 1. According to the temperature of heat treatment, in either case, these are designated as a to m in Table 1. Example "a" is a control example, in each case representing the un-heat treated starting material. Examples b, c, j, k and m are reference examples.

Table 1

| Example | Temp °C | PO$_4$ binding | | Soluble Mg Release | | Surface absorbed water | | Mixed metal species |
|---------|---------|------------|------------|-----------|-----------|-----------|-----------|---------------------|
| | | Batch A % | Batch B % | Batch A mmol/l | Batch B mmol/l | Batch A % | Batch B % | Batch A (peak at 34° θ assigned to Formula I) XRD-counts |
| a | Uncalcined | 51.1 | 43.2 | 7.4 | 8.8 | 3.12 | 4.72 | 0 |
| b | 150 | 50.9 | 46.2 | 7.3 | 7.0 | 1.11 | 0.43 | 0 |
| c | 200 | 49.6 | 55.9 | 6.9 | 4.2 | 0.96 | 0.73 | 0 |
| d | 250 | 57.7 | 51.5 | 3.3 | 3.1 | 0.24 | 0.60 | 2100 |
| e | 300 | 61.7 | 54.8 | 2.0 | 2.6 | 0.52 | 0.46 | 2100 |
| f | 350 | 66.0 | 64.7 | 0.7 | 0.9 | 0.00 | 0.23 | 1700 |
| g | 400 | 65.2 | 61.2 | 0.7 | 0.7 | 0.25 | 0.61 | 1600 |
| h | 450 | 66.0 | 61.8 | 1.5 | 1.1 | 0.00 | 0.82 | 1600 |
| i | 500 | 64.0 | 84.6 | 0.8 | 0.3 | 0.96 | 0.96 | 1600 |

(continued)

| Example | Temp °C | PO$_4$ binding | | Soluble Mg Release | | Surface absorbed water | | Mixed metal species |
|---|---|---|---|---|---|---|---|---|
| | | Batch A % | Batch B % | Batch A mmol/l | Batch B mmol/l | Batch A % | Batch B % | Batch A (peak at 34° θ assigned to Formula I) XRD-counts |
| j | 600 | 42.8 | 58.3 | 6.0 | 8.4 | 1.09 | 1.11 | 0 |
| k | 750 | 28.5 | 45.2 | 10.7 | 14.6 | - | - | 0 |
| m | 1000 | 17.6 | 13.0 | 13.3 | 19.5 | - | - | 0 |

Further examples were prepared as detailed below using further methods for preparation.

**METHOD 2 (M2)**

Two starting materials, designated solution 1 and solution 2 were prepared by the method set out below.

Magnesium sulphate and iron sulphate were dissolved in AnalaR™ water to prepare solution 1. In a separate vessel sodium carbonate and sodium hydroxide were dissolved in AnalaR™ water to prepare solution 2. The weights used were calculated to give the desired ratio of metal cations. The solutions were then added simultaneously over 50 minutes to stirred heel water at controlled flow rates sufficient to maintain pH 10.3 in the reaction mixture (+/- 0.2 pH units) at a reaction temperature not exceeding 30°C. When the additions were complete, the reaction mixture was mixed for another 30 minutes and then filtered using a buchner filtration set up. The filtered product was then washed with 220ml portions of cold AnalaR™ water. After isolation the product was dried using a preheated oven.

For the preparation of solution 1, AnalaR™ water was weighed out into a vessel and stirred using an overhead mixer, into which was dissolved an appropriate amount of iron sulphate solution (Ferripure, ex Kemira). Once dissolved, magnesium sulphate (Epsom Salt, ex William Blythe) was quantitatively transferred to the stirred iron sulphate solution and allowed to dissolve.

For the preparation of solution 2, AnalaR™ water was weighed out into a vessel and stirred using an overhead mixer, into which was dissolved an appropriate amount of sodium carbonate (Pharmakarb, ex Brunner Mond). Once dissolved, sodium hydroxide (Pearl Caustic Soda, ex INEOS Chlor) was quantitatively transferred to the stirred sodium carbonate solution and allowed to dissolve.

A calculated weight of AnalaR™ water was placed into a vessel. Flow control units were used to deliver the appropriate flow rates of the alkaline carbonate and metal sulphate solutions.

After starting the propeller-blade mixer in the vessel containing the water, the feed pumps were started. The alkaline carbonate and metal sulphate solutions then started flowing into the vessel. During the precipitation the pH of the reaction was monitored using a pH-meter. The target pH was 10.3 (+/-0.2 pH units). It tool ca. 5-10 minutes before the pH stabilised within the desired band. The pH was checked at 1 minute intervals during the 50 minute addition time, and the flow rate adjusted of the carbonate stream as necessary to control the pH. The temperature was also checked at 1 minute intervals. The total addition time was 50 minutes. The batch was then stirred for 30 minutes after the addition phase.

The product slurry was then filtered using a vacuum pump and buchner flask with a Whatman™ hardened ashless filter paper (No 541). After filtering, the filter cake was washed with portions of AnalaR™ water. After isolation the washed product was transferred to a vessel and dried in a preheated oven at 120°C.

Product sample for analysis were ground using a ball mill (Retsch PM 100).

Product sample for analysis was milled through a stainless steel, 200mm diameter, 106μm sieve, using a sieve shaker (Retsch AS-200). Oversize material was returned to the stock dried sample to be reground, until all material is < 106μm.

The composition of solutions 1 and 2 used to produce Examples 1 and 3, prepared according to method M2, were the following:

| Example | Solution 1 | Solution 2 |
|---|---|---|
| Example 1 | 570 g MgSO4.7H$_2$O | 277 g NaOH |
| | 552 g Ferric Sulphate solution | 230 g Na2CO3 |
| | 1415 g AnalaR™ water | 1735 g AnalaR™ water |
| Example 2 | 412 g MgSO4.7H$_2$O | 167 g NaOH |

|       |                        |         |                    |
|-------|------------------------|---------|--------------------|
| 199 g | Ferric Sulphate solution | 138 g  | Na2CO3             |
| 928 g | AnalaR™ water          | 1044 g | AnalaR™ water      |

The reaction for example 1 can be described by the following equation:

$$4MgSO_4 + Fe_2(SO_4)_3 + 12\ NaOH + Na_2CO_3 \rightarrow Mg_4Fe_2(OH)_{12}.CO_3.nH_2O + 7Na_2SO_4$$

By changing the ratio of $M^{II}$ : $M^{III}$ cations to 2:1, 3:1, 4:1 different composition materials were achieved.

**Method 3 (M3)**

Preparation of the product was as for the method detailed in M2 except that the two solutions were pumped into a flask with an overflow at ~ 2 litres and constantly mixing.
After discarding the first litre, 3-4 litres of overflowing slurry was collected.

**Method 4 (M4)**

Preparation of the product was performed as for the method detailed in M2, up to the addition of solutions 1 and 2. On production of the slurry and subsequent filtering no washing process was performed on the product. After isolation the unwashed product was transferred to a vessel and dried in a preheated oven at 120°C.

**Method 5 (M5)**

Preparation of a 4:1 ratio material was performed as for the method detailed in M2, up to the addition of solutions A and B. On production of the slurry the batch was split into two halves. One half of the slurry was treated as for M2 method, which involved filtering using buchner filtration equipment, washed with the required AnalaR™ water and dried in a pre heated oven at 120°C. The second half of the slurry was subjected to an aging process involving transferred to a glass beaker and heated on a heating mantle at 60°C for 4 hours. The product was the filtered, washed and dried as for M2. The aged sample was then used as example 5.

**Method 6 (M6)**

Example 6 was a commercial MgAl mixed metal compound (Macrosorb CT-100) sourced from Ineos Silicas Ltd UK.

**Method (M7)**

This route involved anionic exchange with chloride ions.
To a stirred slurry of 3:1 ratio material (prepared by method M3), a solution of Hydrochloric acid was added over 20 minutes at a controlled flow rate sufficient to maintain pH 9.5 to 10.5 in the reaction mixture. This addition was performed under a nitrogen atmosphere, achieved by purging the slurry throughout the addition step with a nitrogen stream. When the addition was complete the reaction mixture was stirred for another 5 minutes with nitrogen purging, and then filtered using a buchner filtration set up. The filtered product was then washed with 9 x 220ml portions of cold AnalaR™ water. After isolation the product was dried using a preheated oven.
For the preparation of the stock slurry, 22g of 3:1 ratio material was weighed out and quantitatively transferred into a vessel containing 200ml AnalaR™ water stirred using an overhead mixer. The resulting slurry was subjected to continuous nitrogen purging.
For the preparation of Hydrochloric acid solution, a 1 M stock solution was diluted to give a suitable 0.05M solution. The solution was purged with nitrogen for 30 minutes prior to use.
After starting the overhead mixer in the vessel containing the stock slurry, the feed pump was started introducing the hydrochloric acid solution into the vessel. During the addition phase, the pH of the reaction and temperature was monitored using a pH-meter. The total addition time was 20 minutes. After the addition phase the batch was then stirred for 5 minutes with continuous nitrogen purging.
The product slurry was then filtered using a vacuum pump and buchner flask with a Whatman™ hardened ashless filter paper (No 541). After filtering, the filter cake was washed with 9 portions of 220ml AnalaR™ water. After isolation the washed product was transferred to a vessel and dried in a preheated oven at 120°C.

Product sample for analysis were ground using a ball mill (Retsch PM 100).

Product sample for analysis was milled through a stainless steel, 200mm diameter, $106\mu$m sieve, using a sieve shaker (Retsch AS-200). Oversize material was returned to the stock dried sample to be reground, until all material is < $106\mu$m.

### Test Method 4 Carbonate Analysis

A sample of known mass is combusted at around 1350°C in a furnace in a pure oxygen atmosphere. Any carbon in the sample is converted to $CO_2$ which is passed through a moisture trap before being measured by an infra-red detector. By comparing against a standard of known concentration, the carbon content of the sample can be found.

A Leco SC-144DR Carbon and Sulphur Analyser, with oxygen supply, ceramic combustion boats, boat lance and tongs was used.

0.2g (+/- 0.01 g) of sample was weighed into a combustion boat. The boat was then placed into the Leco furnace and the carbon content analysed.

The analysis was performed in duplicate.

The % carbonate was determined by:

$$\%C \text{ (sample)} = (\%C_1 + \%C_2) / 2$$

where $C_1$ and $C_2$ are individual carbon results.

$$\%CO3 = \%C \times 60/12.$$

### Test Method 5 XRF analysis

XRF analysis of the product was performed by using a Philips PW2400 Wavelength Dispersive XRF Spectrometer. The sample was fused with 50:50 lithium tetra/metaborate (high purity) and presented to the instrument as a glass like bead.

All reagents used are analytical grade or equivalent unless specified. AnalaR™ water, Lithium tetraborate 50% metaborate 50% flux (high Purity Grade ICPH Fluore-X 50),

A muffle Furnace capable of 1025 °C, extended tongs, hand tongs, Pt/5%Au casting tray and Pt/5%/Au dish were used.

1.5g (+/- 0.0002g) of sample and 7.5000g (+/- 0.0002g) of tetra/metaborate was accurately weighed out into a Pt/5%/Au dish. The two constituents were lightly mixed in the dish using a spatula, prior to placement in the furnace preset to 1025°C for 12minutes. The dish was agitated at 6 minutes and 9 minutes to ensure homogeneity of the sample. Also at 9 minutes the casting tray was placed in the furnace to allow for temperature equlibriation. After 12 minutes the molten sample was poured into the casting tray, which was removed from the furnace and allowed to cool. The bead composition was determined using the spectrophotometer.

Details of the $M^{II}$:$M^{III}$ ratios and methods of preparation are detailed in Table 2 for the original samples A and B as, and for the further samples 1 to 7

Table 2

| | | Compositions of metal compounds used for calcination experiments | | |
|---|---|---|---|---|
| Example | | Molar Ratio $M^{II}$:$M^{III}$ | Anion Type | Method of preparation |
| A | Mg and Fe | 2 | carbonate | M1 |
| B | Mg and Fe | 4 | carbonate | M1 |
| 1 | Mg and Fe | 2 | carbonate | M2 |
| 2 | Mg and Fe | 3 | carbonate | M3 |
| 3 | Mg and Fe | 4 | carbonate | M2 |
| 4 | Mg and Fe | 2 | carbonate | M4 |

(continued)

| Compositions of metal compounds used for calcination experiments | | | | |
|---|---|---|---|---|
| Example | | Molar Ratio M$^{II}$:M$^{III}$ | Anion Type | Method of preparation |
| | | | | |
| 5 | Mg and Fe | 4 | carbonate | M5 |
| 6 | Mg and Al | 3 | carbonate | M6 |
| 7 | Mg and Fe | 3 | chlorine | M7 |

Table 3 shows the compositional analysis results (nominally expressed as oxide percentages) for the examples for uncalcined and for calcined materials.

In Tables 3 and 4, samples calcined for greater than 3 hours, or calcined at a temperature outside 250 to 500 °C are reference examples.

Table 3

| Start batch | Temp °C | calcination time | percent weight expressed as oxide | | | | | | |
|---|---|---|---|---|---|---|---|---|---|
| | | | %Na2O | %MgO | %Fe2O3 | %H2O | %CO2 | %SO3 | %CO3 |
| | | | | | | | | | |
| A | u | | 1.98 | 29.71 | 28.39 | 28.92 | 7.97 | 3.03 | 10.9 |
| A | 300 | 3 hrs | 2.44 | 35.49 | 33.74 | 17.88 | 7.79 | 2.66 | 10.6 |
| A | 500 | 3 hrs | 1.64 | 42.28 | 41.78 | 6.19 | 1.87 | 6.25 | 2.6 |
| A | 600 | 3 hrs | 3.19 | 45.81 | 43.64 | 3.65 | 0.36 | 3.36 | 0.5 |
| A | 1000 | 3 hrs | 3.25 | 47.14 | 45.08 | 0.00 | 0.00 | 4.61 | 0.0 |
| B | u | | 1.74 | 37.06 | 18.64 | 31.40 | 8.02 | 3.14 | 10.9 |
| B | 300 | 3 hrs | | | | | | | |
| B | 500 | 3 hrs | | | | | 1.89 | | 2.6 |
| B | 600 | 3 hrs | | | | | 0.24 | | 0.3 |
| B | 1000 | 3 hrs | | | | | 0.00 | | 0.0 |
| 1 | u | | 0.05 | 28.08 | 28.72 | 34.82 | 6.47 | 1.86 | 8.8 |
| 1 | 300 | 3 has | 0.05 | 30.82 | 31.07 | 29.54 | 6.60 | 1.92 | 9.0 |
| 1 | 500 | 3 hrs | 0.05 | 37.23 | 38.00 | 20.96 | 1.11 | 2.65 | 1.5 |
| 1 | 750 | 3 hrs | | | | | 0.00 | | 0.0 |
| 2 | u | | 0.05 | 32.78 | 22.43 | 36.47 | 6.82 | 1.45 | 9.3 |
| 2 | 500 | 3 hrs | 0.05 | 53.81 | 36.63 | 5.69 | 0.51 | 3.31 | 0.7 |
| 3 | u | | 0.05 | 37.26 | 19.31 | 34.07 | 6.99 | 2.32 | 9.5 |
| 3 | 200 | 3 hrs | 0.05 | 43.63 | 22.64 | 22.77 | 8.17 | 2.74 | 11.1 |
| 3 | 500 | 3 hrs | 0.05 | 59.12 | 31.03 | 5.00 | 1.00 | 3.80 | 1.4 |
| 4 | u | | 20.91 | 17.70 | 17.21 | 15.77 | 7.85 | 20.56 | 10.7 |
| 4 | 300 | 3 hrs | 22.35 | 20.13 | 19.10 | 8.50 | 8.04 | 21.88 | 11.0 |
| 4 | 500 | 3 hrs | 26.75 | 23.17 | 22.44 | 4.16 | 3.28 | 20.20 | 4.5 |
| 4 | 750 | 3 hrs | | | | | 0.83 | | 1.1 |
| 5 | u | | 0.05 | 40.14 | 20.70 | 29.39 | 7.98 | 1.74 | 10.9 |
| 5 | 200 | 3 hrs | 0.05 | 42.91 | 21.89 | 24.56 | 8.75 | 1.84 | 11.9 |

(continued)

| Start batch | Temp °C | calcination time | percent weight expressed as oxide | | | | | | |
|---|---|---|---|---|---|---|---|---|---|
| | | | %Na2O | %MgO | %Fe2O3 | %H2O | %CO2 | %SO3 | %CO3 |
| 5 | 500 | 3 hrs | 0.05 | 57.01 | 29.54 | 9.00 | 1.93 | 2.48 | 2.6 |
| 6 | u | | | | | | | | |
| 6 | 300 | 3 hrs | | | | | | | |
| 6 | 500 | 3 hrs | | | | | | | |
| 6 | 750 | 3 hrs | | | | | | | |
| 7 | u | | 0.05 | 35.62 | 24.85 | | | | |
| 7 | 500 | 3 hrs | | | | | | | |
| 7 | 750 | 3 hrs | | | | | | | |
| 2 | 500 | 5 min | 0.09 | 43.34 | 42.84 | 8.94 | 2.09 | 2.70 | 2.8 |
| 2 | 500 | 16 hrs | 1.34 | 45.41 | 44.83 | 4.33 | 1.25 | 2.84 | 1.7 |
| u= uncalcined material | | | | | | | | | |

Table 4

| | Temp °C | Calc. time | Formula constants $[M^{II}(1-a)M^{III}(a)O_bA^{n-}{}_c.zH2O]$ | | | | | PO4 bound %weight | Mg released mmol/l |
|---|---|---|---|---|---|---|---|---|---|
| | | | a | b | c | z | $\sum cn/a$ | | |
| A | u | | x0.33 | - | y0.17 | m1.47 | - | 51.1 | 7.4 |
| A | 300 | 3 hrs | 0.32 | 1.03 | 0.14 | 0.76 | 0.84 | 61.7 | 2.0 |
| A | 500 | 3 hrs | 0.33 | 1.11 | 0.06 | 0.22 | 0.34 | 64.0 | 0.8 |
| A | 600 | 3 hrs | 0.32 | 1.15 | 0.01 | 0.12 | 0.07 | 42.8 | 6.0 |
| A | 1000 | 3 hrs | 0.33 | 1.15 | 0.01 | 0.00 | 0.09 | 17.6 | 13.3 |
| B | u | | x0.20 | - | y0.16 | - | - | 43.2 | 8.8 |
| B | 300 | 3 hrs | | | | | | 54.8 | 2.6 |
| B | 500 | 3 hrs | | | | | | 84.6 | 0.3 |
| B | 600 | 3 hrs | | | | | | 58.3 | 8.4 |
| B | 1000 | 3 hrs | | | | | | 13.0 | 19.5 |
| 1 | u | | x0.34 | - | y0.14 | m1.83 | - | 67.6 | 6.0 |
| 1 | 300 | 3 hrs | 0.34 | 1.04 | 0.13 | 1.42 | 0.77 | 73.2 | 0.2 |
| 1 | 500 | 3 | 0.34 | 1.15 | 0.02 | 0.83 | 0.11 | 80.0 | 0.4 |
| 1 | 750 | 3 hrs | | | | | | 51.0 | 12.1 |
| 2 | u | | x0.26 | - | y0.14 | m1.85 | - | 59.7 | 7.8 |
| 2 | 500 | 3 hrs | 0.26 | 1.12 | 0.01 | 0.18 | 0.09 | 79.4 | 0.3 |
| 3 | u | | x0.21 | - | y0.14 | m1.62 | - | 51.7 | 5.9 |
| 3 | 200 | 3 hrs | 0.21 | 0.97 | 0.14 | 0.93 | 1.33 | 64.8 | 1.7 |
| 3 | 500 | 3 hrs | 0.21 | 1.08 | 0.02 | 0.15 | 0.20 | 91.3 | 0.1 |
| 4 | u | | x0.33 | - | y0.62 | m1.34 | - | 28.3 | 2.8 |

(continued)

|  | Temp | Calc. | Formula constants [$M^{II}(1-a)M^{III}(a)O_bA^{n-}_c.zH_2O$] |  |  |  |  | PO4 bound | Mg released |
|---|---|---|---|---|---|---|---|---|---|
|  | °C | time |  |  |  |  |  | %weight | mmol/l |
| 4 | 300 | 3 hrs | 0.32 | 0.59 | 0.57 | 0.64 | 3.55 | 30.7 | 3.5 |
| 4 | 500 | 3 hrs | 0.33 | 0.82 | 0.34 | 0.27 | 2.10 | 17.1 | 1.4 |
| 4 | 750 | 3 hrs |  |  |  |  |  | 10.6 | 0.5 |
| 5 | u |  | x0.21 | - | y0.14 | m1.30 | - | 66.2 | 3.4 |
| 5 | 200 | 3 hrs | 0.20 | 0.95 | 0.15 | 1.02 | 1.45 | 67.6 | 1.6 |
| 5 | 500 | 3 hrs | 0.21 | 1.08 | 0.02 | 0.28 | 0.24 | 87.1 | 0.0 |
| 6 | u |  |  | - |  |  |  | 42.8 | 8.13 |
| 6 | 300 | 3 hrs |  |  |  |  |  | 47.6 | 3.32 |
| 6 | 500 | 3 hrs |  |  |  |  |  | 94.2 | 0 |
| 6 | 750 | 3 hrs |  |  |  |  |  | 71.7 | 1.87 |
| 7 | u |  | x0.26 | - |  |  | - | 44.3 | 6.55 |
| 7 | 500 | 3 hrs |  |  |  |  |  | 72.6 | 0.36 |
| 7 | 750 | 3 hrs |  |  |  |  |  | 64.1 | 0.71 |
| 2 | 500 | 5 min | 0.33 | 1.14 | 0.03 | 0.31 | 0.18 | 84.6 | 0.15 |
| 2 | 500 | 16 hrs | 0.33 | 1.15 | 0.02 | 0.14 | 0.10 | 73.5 | 1.05 |
| u=uncalcined |  |  |  |  |  |  |  |  |  |

Note that for the uncalcined material, which is in the form of formula (II), values of x, y and m have been indicated in the table in place of a, c and z.

In Table 4, % $H_2O$ is calculated as follows:

$$\% \ H_2O = 100\% - (\%Na_2O \ \%MgO + \%Fe_2O_3 + \%SO_3 \ ^+ \%CO_2$$

By % is meant %weight/weight. $\%Na_2O \ \%MgO + \%Fe_2O + \%SO_3$ was determined by XRF $\%CO_2$ determined by LECO Carbon analysis

$$\%CO_2 = (Mw \ CO_2/MwCO_3) \times (\%CO_3) = 44/60* \ \%CO_3$$

The values for a, b, c and z were derived in Table 4 from the values in Table 3 as follows (in the calculations, $M_w$ denotes molecular weight.

Calculation of a

$$a = \frac{moles M^{III}}{(moles M^{II} + moles M^{III})}$$

Substituting molar fractions in this formula gives

$$a = \frac{(2 \times \%Fe_2O_3 / MwFe_2O_3)}{((\%MgO / MwMgO) + (2 \times \%Fe_2O_3 / MwFe_2O_3))}$$

Mw $Fe_2O_3$ 159.6

Mw MgO = 40.3
%Fe$_2$O$_3$ and %MgO values from table 4

Calculation of c

Molar fraction trivalent metal (Fe$^{III}$) divided by molar fraction anion (CO$_3$ = a divided by c.

$$\left(2 \times \%Fe_2O_3 / MwFe_2O_3\right) \Big/ \left(\%CO_3 / MwCO_3\right) = a/c$$

Substituting value for a and molecular weights and %w/w (table 3) for Fe$_2$O$_3$ and CO$_3$ in above equation gives c.

$$c = \frac{0.33 \times \left(\%CO_3 / MwCO_3\right)}{\left(2 \times \%Fe_2O_3 / MwFe_2O_3\right)}$$

Molar fraction of SO$_4$ available as interlayer anion was calculated by subtracting from the total amount of SO$_4$ the amount of SO$_4$ which would be associated with the Na$_2$O in the form of Na$_2$SO$_4$. The value for exchangeable SO$_4$ was then added to the mole fraction of that for the carbonate anion in the calculation above.

Calculation of b

b is calculated by assuming that formula [M$^{II}$$_{(1-a)}$M$^{III}$$_{(a)}$O$_b$A$^{n-}$$_c$.zH$_2$O] is charge-neutral.
This means that the total of product of charge associated with each element and constants a, b, c should be zero resulting in the following equation:

$$2(1-a) + 3a + (-2b) + (-nc) = 0$$

this can be rewritten as:

$$b = \left(2 + a - nc\right) \Big/ 2$$

substituting n = 2 (charge for the carbonate anion) and value for a and c into above formula gives b.

Calculation of z

Molar fraction trivalent metal (Fe$^{III}$) divided by molar fraction H$_2$O = a divided by z.

$$\left(2 \times \%Fe_2O_3 / MwFe_2O_3\right) \Big/ \left(\%H_2O / MwH_2O\right) = a/z$$

Substituting a and molecular weight and %w/w (table 4) for H$_2$O and Fe$_2$O$_3$ in above equation gives z.

$$z = \frac{0.33 \times \left(\%H_2O / MwH_2O\right)}{\left(2 \times \%Fe_2O_3 / MwFe_2O_3\right)}$$

Calculation of $\sum cn/a$

Calculated by substituting c, n and a values.

pH Effect

Table 6 below shows the effect of pH of the phosphate solution on the binding and Magnesium release.

Table 6

| Phosphate binder | Calcination Temp | PO4 bound | PO4 bound | Mg release | Mg release |
|---|---|---|---|---|---|
| | | pH = 4 | pH=7 | pH = 4 | pH = 7 |
| | | % | % | mmol/l | mmol/l |
| Example 1 | 500°C for 3 hrs | 81 | 79 | 0.07 | 0.06 |
| Example 1 | uncalcined | 56 | 57 | 0.32 | 0.16 |

The results from table 6 were obtained by the phosphate binding test as described in test method 2 but with the following changes: 0.5 g of the phosphate binder was dissolved in 125 ml 4 mmol/l phosphate solution. The samples were then incubated in stoppered polypropylene conical flasks in a shaking water bath at 37°C and 200rpm for 30 minutes.

**Claims**

1. A substance for use as a medicament, comprising a phosphate binding, solid mixed metal compound of formula (I):

$$M^{II}_{1-a}M^{III}_aO_bA^{n-}_c.zH_2O \qquad (I)$$

where $M^{II}$ is at least one bivalent metal; $M^{III}$ is $Fe^{3+}$; $A^{n-}$ is at least one n-valent anion; a = moles of $M^{III}$/ (moles of $M^{II}$ + moles of $M^{III}$); 2+a = 2b+$\sum cn$ and $\sum cn$ < 0.9a, wherein the compound of formula (I) is obtainable by heating a starting material comprising a layered double hydroxide structure at a temperature in a range of 250°C to 500°C for 3 hours or less.

2. A substance for use as a medicament according to claim 1, wherein in formula (I), a is $\geq$ 0.3.

3. A substance for use as a medicament according to claim 1, wherein in formula (I), a is < 0.3.

4. A substance for use as a medicament according to claim 2, wherein in formula (I), 0.03a< $\sum cn$ < 0.5a.

5. A substance for use as a medicament according to claim 3, wherein in formula (I), 0.03a < $\sum cn$ < 0.7a.

6. A substance for use as a medicament according to any preceding claim, wherein the solid mixed metal compound has less than 2%, preferably less than 1.5%, more preferably less than 1% by weight crystallite-surface absorbed water.

7. A substance for use as a medicament, comprising a solid mixed metal compound obtained or obtainable by heating a starting material comprising a layered double hydroxide structure at a temperature of from 250°C to 500°C for 3 hours or less.

8. A substance for use as a medicament according to claim 7, wherein the starting material comprises a compound of formula (II):

$$M^{II}_{1-x}M^{III}_x(OH)_2A^{n-}_y.mH_2O \qquad (II)$$

where $M^{II}$ is at least one bivalent metal; $M^{III}$ is at least one trivalent metal; $A^{n-}$ is at least one n-valent anion; x = $\sum yn$, 0 < x $\leq$ 0.5, 0 < y $\leq$ 1 and 0 < m $\leq$ 10.

9.  A substance for use as a medicament according to claim 8, wherein in formula (II), x is $\geq 0.3$.

10. A substance for use as a medicament according to claim 8, wherein in formula (II), x is < 0.3.

11. A substance for use as a medicament according to any of claims 7 to 10, wherein the substance has a 10% higher phosphate binding capacity relative to the compound of formula (II) from which it is obtainable.

12. Use in the manufacture of a medicament for binding of phosphate in an animal in need thereof of a substance comprising a phosphate binding, solid mixed metal compound of formula (I):

$$M^{II}_{1-a}M^{III}_aO_bA^{n-}_c.zH_2O \qquad (I)$$

where $M^{II}$ is at least one bivalent metal; $M^{III}$ is $Fe^{3+}$; $A^{n-}$is at least one n-valent anion; a = moles of $M^{III}$/ (moles of $M^{II}$ + moles of $M^{III}$); $2+a = 2b+\sum cn$ and $\sum cn < 0.9a$, wherein the compound of formula (I) is obtainable by heating a starting material comprising a layered double hydroxide structure at a temperature in a range of 250°C to 500°C for 3 hours or less.

13. Use in the manufacture of a medicament for prophylaxis or treatment of hyperphosphataemia, of a substance comprising a phosphate binding, solid mixed metal compound of formula (I):

$$M^{II}_{1-a}M^{III}_aO_bA^{n-}_c.zH_2O \qquad (I)$$

where $M^{II}$ is at least one bivalent metal; $M^{III}$ is $Fe^{3+}$; $A^{n-}$ is at least one n-valent anion; a = moles of $M^{III}$/ (moles of $M^{II}$ + moles of $M^{III}$); $2+a = 2b+\sum cn$ and $\sum cn < 0.9a$, wherein the compound of formula (I) is obtainable by heating a starting material comprising a layered double hydroxide structure at a temperature in a range of 250°C to 500°C for 3 hours or less.

14. Use, according to claim 12 or claim 13, wherein in formula (I), a is $\geq 0.3$ and preferably $0.03a < \sum cn < 0.5a$.

15. Use according to claim 12 or claim 13, wherein in formula (I), a is < 0.3 and preferably $0.03a < \sum cn < 0.7a$.

16. Use in the manufacture of a medicament, for binding of phosphate in an animal in need thereof, of a substance obtainable by heating a starting material comprising a layered double hydroxide structure at a temperature of from 250°C to 500°C for three hours or less.

17. Use in the manufacture of a medicament, for prophylaxis or treatment of hyperphosphataemia, of a substance obtainable by heating a starting material comprising a layered double hydroxide structure at a temperature of from 250°C to 500°C for three hours or less.

18. Use according to claim 16 or claim 17, wherein the starting material comprises a compound of formula (II):

$$M^{II}_{1-x}M^{III}_{a(}OH)_2A^{n-}_y.zH_2O \qquad (II)$$

where $M^{II}$ is at least one bivalent metal; $M^{III}$ is at least one trivalent metal; An- is at least one n-valent anion; $x=\sum ny$; and $0 < x \leq 0.5$; $0 \leq m \leq 10$.

19. Use, according to claim 18, wherein in formula (II), x is $\geq 0.3$.

20. Use according to claim 18, wherein in formula (II), x is < 0.3.

21. A method of preparing a substance for use as a medicament, the method comprising heating a starting material comprising a layered double hydroxide structure at a temperature of from 250°C to 500°C for three hours or less.

22. A method according to claim 21, wherein the starting material comprises a compound of formula (II):

$$M^{II}_{1-x}M^{III}_a(OH)_2A^{n-}_y.mH_2O \qquad (II)$$

where $M^{II}$ is at least one bivalent metal; $M^{III}$ is at least one trivalent metal; $A^{n-}$ is at least one n-valent anion; $x=\sum ny$;

and $0 < x \leq 0.5$, $0 \leq m \leq 10$.

23. A method according to claim 22, wherein in formula (II), x is $\geq 0.3$.

24. A method according to claim 22, wherein in formula (II), x is < 0.3.

25. A method according to any of claims 21 to 24, wherein the starting material is substantially free of particles greater than $100\mu m$.

26. A pharmaceutical formulation comprising: a phosphate binding solid mixed metal compound of formula (I):

$$M^{II}_{1-a}M^{III}_aO_bA^{n-}_c.zH_2O \qquad (I)$$

where $M^{II}$ is at least one bivalent metal; $M^{III}$ is $Fe^{3+}$; $A^{n-}$ is at least one n-valent anion; a = moles of $M^{III}$/ (moles of $M^{II}$ + moles of $M^{III}$); $2+a = 2b+\sum cn$ and $\sum cn < 0.9a$; and a pharmaceutically acceptable carrier, wherein the compound of formula (I) is obtainable by heating a starting material comprising a layered double hydroxide structure at a temperature in a range of 250°C to 500°C for 3 hours or less.

27. A pharmaceutical formulation according to claim 26, wherein in formula (I), a is $\geq 0.3$ and preferably $0.03a < \sum cn < 0.5a$.

28. A pharmaceutical formulation according to claim 26, wherein in formula (I), a is < 0.3 and preferably $0.03a < \sum cn < 0.7a$.

29. A pharmaceutical formulation comprising a solid mixed metal compound obtainable by heating a starting material comprising a layered double hydroxide structure at a temperature of from 250°C to 500°C for three hours or less, and a pharmaceutically acceptable carrier.

30. A pharmaceutical formulation according to claim 29, wherein the starting material comprises a compound of formula (II):

$$M^{II}_{1-x}M^{III}_x(OH)_2A^{n-}_y.mH_2O \qquad (II)$$

where $M^{II}$ is at least one bivalent metal; $M^{III}$ is at least one trivalent metal; $A^{n-}$ is at least one n-valent anion; $x=\sum ny$; and $0 < x \leq 0.5$, $0 \leq m \leq 10$.

31. A pharmaceutical formulation according to claim 30, wherein in formula (II), x is $\geq 0.3$.

32. A pharmaceutical formulation according to claim 30, wherein in formula (II), x is < 0.3.

33. A method of preparing a pharmaceutical formulation according to any of claims 26 to 32, the method comprising admixing the phosphate binding solid mixed metal compound, the pharmaceutically acceptable carrier and optionally, any other ingredients.

**Patentansprüche**

1. Stoff für die Verwendung als Medikament, umfassend eine phosphatbindende, feste Mischmetallverbindung der Formel (I):

$$M^{II}_{1-a}M^{III}_aO_bA^{n-}_c.zH_2O \qquad (I),$$

wobei $M^{II}$ wenigstens ein zweiwertiges Metall ist; $M^{III}$ $Fe^{3+}$ ist; $A^{n-}$ wenigstens ein n-wertiges Anion ist; a = mol $M^{III}$/ (mol $M^{II}$ + mol $M^{III}$) ; $2 + a = 2b + \sum cn$ und $\sum cn < 0.9a$, wobei die Verbindung der Formel (I) durch Wärmen eines Ausgangsmaterials, das eine Schichtdoppelhydroxid-Struktur umfasst, bei einer Temperatur in dem Bereich von 250 °C bis 500 °C für 3 Stunden oder weniger erhältlich ist.

2. Stoff für die Verwendung als Medikament gemäß Anspruch 1, wobei in der Formel (I) a $\geq 0.3$ ist.

**3.** Stoff für die Verwendung als Medikament gemäß Anspruch 1, wobei in der Formel (I) a < 0,3 ist.

**4.** Stoff für die Verwendung als Medikament gemäß Anspruch 2, wobei in der Formel (I) 0,03a < $\sum cn$ < 0,5a.

**5.** Stoff für die Verwendung als Medikament gemäß Anspruch 3, wobei in der Formel (I) 0,03a < $\sum cn$ < 0,7a.

**6.** Stoff für die Verwendung als Medikament gemäß einem der vorstehenden Ansprüche, wobei die feste Mischmetallverbindung weniger als 2 %, vorzugsweise weniger als 1,5 %, bevorzugter weniger als 1 Gew. -% an Kristallitoberflächenabsorbiertem Wasser aufweist.

**7.** Stoff für die Verwendung als Medikament, umfassend eine feste Mischmetallverbindung, erhalten oder erhältlich durch Wärmen eines Ausgangsmaterials, das eine Schichtdoppelhydroxid-Struktur umfasst, bei einer Temperatur von 250 °C bis 500 °C für 3 Stunden oder weniger.

**8.** Stoff für die Verwendung als Medikament gemäß Anspruch 7, wobei das Ausgangsmaterial eine Verbindung der Formel (II) umfasst:

$$M^{II}_{1-x}M^{III}_x(OH)_2A^{n-}_y.mH_2O \qquad (II),$$

wobei $M^{II}$ wenigstens ein zweiwertiges Metall ist; $M^{III}$ wenigstens ein dreiwertiges Metall ist; $A^{n-}$ wenigstens ein n-wertiges Anion ist; x = $\sum yn$, 0 < x $\leq$ 0,5, 0 < y $\leq$ 1 und 0 < m $\leq$ 10.

**9.** Stoff für die Verwendung als Medikament gemäß Anspruch 8, wobei in der Formel (II) x $\geq$ 0,3 ist.

**10.** Stoff für die Verwendung als Medikament gemäß Anspruch 8, wobei in der Formel (II) x < 0,3 ist.

**11.** Stoff für die Verwendung als Medikament gemäß einem der Ansprüche 7 bis 10, wobei der Stoff eine 10 % höhere Phosphatbindungskapazität aufweist als die Verbindung der Formel (II), aus der er erhältlich ist.

**12.** Verwendung eines Stoffs, der eine phosphatbindende, feste Mischmetallverbindung der Formel (I) umfasst, bei der Herstellung eines Medikaments zum Binden von Phosphat in einem Tier mit Bedarf daran:

$$M^{II}_{1-a}M^{III}_aO_bA^{n-}_c. zH_2O \qquad (I),$$

wobei $M^{II}$ wenigstens ein zweiwertiges Metall ist; $M^{III}$ $Fe^{3+}$ ist; $A^{n-}$ wenigstens ein n-wertiges Anion ist; a = mol $M^{III}$/(mol $M^{II}$ + mol $M^{III}$) ; 2 + a = 2b + $\sum cn$ und $\sum cn$ < 0,9a, wobei die Verbindung der Formel (I) durch Wärmen eines Ausgangsmaterials, das eine Schichtdoppelhydroxid-Struktur umfasst, bei einer Temperatur in dem Bereich von 250 °C bis 500 °C für 3 Stunden oder weniger erhältlich ist.

**13.** Verwendung eines Stoffs, der eine phosphatbindende, feste Mischmetallverbindung der Formel (I) umfasst, bei der Herstellung eines Medikaments für die Vorbeugung oder Behandlung von Hyperphosphatämie:

$$M^{II}_{1-a}M^{III}_aO_bA^{n-}_c.zH_2O \qquad (I),$$

wobei $M^{II}$ wenigstens ein zweiwertiges Metall ist; $M^{III}$ $Fe^{3+}$ ist; $A^{n-}$ wenigstens ein n-wertiges Anion ist; a = mol $M^{III}$/(mol $M^{II}$ + mol $M^{III}$) ; 2 + a = 2b + $\sum cn$ und $\sum cn$ < 0,9a, wobei die Verbindung der Formel (I) durch Wärmen eines Ausgangsmaterials, das eine Schichtdoppelhydroxid-Struktur umfasst, bei einer Temperatur in dem Bereich von 250 °C bis 500 °C für 3 Stunden oder weniger erhältlich ist.

**14.** Verwendung gemäß Anspruch 12 oder Anspruch 13, wobei in der Formel (I) a $\geq$ 0,3 ist und vorzugsweise 0,03a < $\sum cn$ < 0,5a.

**15.** Verwendung gemäß Anspruch 12 oder Anspruch 13, wobei in der Formel (I) a < 0,3 ist und vorzugsweise 0,03a < $\sum cn$ < 0,7a.

**16.** Verwenden eines Stoffs, der durch Wärmen eines Ausgangsmaterials, das eine Schichtdoppelhydroxid-Struktur umfasst, bei einer Temperatur von 250 °C bis 500 °C für drei Stunden oder weniger erhältlich ist, bei der Herstellung eines Medikaments zum Binden von Phosphat in einem Tier mit Bedarf daran.

**17.** Verwenden eines Stoffs, der durch Wärmen eines Ausgangsmaterials, das eine Schichtdoppelhydroxid-Struktur umfasst, bei einer Temperatur von 250 °C bis 500 °C für drei Stunden oder weniger erhältlich ist, bei der Herstellung eines Medikaments für die Vorbeugung oder Behandlung von Hyperphosphatämie.

**18.** Verwendung gemäß Anspruch 16 oder Anspruch 17, wobei das Ausgangsmaterial eine Verbindung der Formel (II) umfasst:

$$M^{II}_{1-x}M^{III}_{x}(OH)_{2}A^{n-}_{y}.mH_2O \qquad (II),$$

wobei $M^{II}$ wenigstens ein zweiwertiges Metall ist; $M^{III}$ wenigstens ein dreiwertiges Metall ist; $A^{n-}$ wenigstens ein n-wertiges Anion ist; $x = \sum ny$; und $0 < x \leq 0,5$; $0 \leq m \leq 10$.

**19.** Verwendung gemäß Anspruch 18, wobei in der Formel (II) $x \geq 0,3$ ist.

**20.** Verwendung gemäß Anspruch 18, wobei in der Formel (II) $x < 0,3$ ist.

**21.** Verfahren zum Herstellen eines Stoffs für die Verwendung als Medikament, wobei das Verfahren das Wärmen eines Ausgangsmaterials, das eine Schichtdoppelhydroxid-Struktur umfasst, bei einer Temperatur von 250 °C bis 500 °C für drei Stunden oder weniger umfasst.

**22.** Verfahren gemäß Anspruch 21, wobei das Ausgangsmaterial eine Verbindung der Formel (II) umfasst:

$$M^{II}_{1-x}M^{III}_{x}(OH)_{2}A^{n-}_{y}.mH_2O \qquad (II),$$

wobei $M^{II}$ wenigstens ein zweiwertiges Metall ist; $M^{III}$ wenigstens ein dreiwertiges Metall ist; $A^{n-}$ wenigstens ein n-wertiges Anion ist; $x = \sum ny$; und $0 < x \leq 0,5$, $0 \leq m \leq 10$.

**23.** Verfahren gemäß Anspruch 22, wobei in der Formel (II) $x \geq 0,3$ ist.

**24.** Verfahren gemäß Anspruch 22, wobei in der Formel (II) $x < 0,3$ ist.

**25.** Verfahren gemäß einem der Ansprüche 21 bis 24, wobei das Ausgangsmaterial im Wesentlichen frei von Partikeln ist, die größer als 100 $\mu$m sind.

**26.** Pharmazeutische Formulierung, umfassend: eine phosphatbindende, feste Mischmetallverbindung der Formel (I):

$$M^{II}_{1-a}M^{III}_{a}O_{b}A^{n-}_{c}.zH_2O \qquad (I),$$

wobei $M^{II}$ wenigstens ein zweiwertiges Metall ist; $M^{III}$ $Fe^{3+}$ ist; $A^{n-}$ wenigstens ein n-wertiges Anion ist; $a = $ mol $M^{III}/$ (mol $M^{II}$ + mol $M^{III}$) ; $2 + a = 2b + \sum cn$ und $\sum cn < 0,9a$; und einen pharmazeutisch verträglichen Träger, wobei die Verbindung der Formel (I) durch Wärmen eines Ausgangsmaterials, das eine Schichtdoppelhydroxid-Struktur umfasst, bei einer Temperatur in dem Bereich von 250 °C bis 500 °C für 3 Stunden oder weniger erhältlich ist.

**27.** Pharmazeutische Formulierung gemäß Anspruch 26, wobei in der Formel (I) $a \geq 0,3$ ist und vorzugsweise $0,03a < \sum cn < 0,5a$.

**28.** Pharmazeutische Formulierung gemäß Anspruch 26, wobei in der Formel (I) $a < 0,3$ ist und vorzugsweise $0,03a < \sum cn < 0,7a$.

**29.** Pharmazeutische Formulierung, umfassend eine feste Mischmetallverbindung, die durch Wärmen eines Ausgangsmaterials, das eine Schichtdoppelhydroxid-Struktur umfasst, bei einer Temperatur von 250 °C bis 500 °C für drei Stunden oder weniger erhältlich ist, und einen pharmazeutisch verträglichen Träger.

**30.** Pharmazeutische Formulierung gemäß Anspruch 29, wobei das Ausgangsmaterial eine Verbindung der Formel (II) umfasst:

$$M^{II}_{1-x}M^{III}_{x}(OH)_{2}A^{n-}_{y}.mH_2O \qquad (II),$$

wobei M$^{II}$ wenigstens ein zweiwertiges Metall ist; M$^{III}$ wenigstens ein dreiwertiges Metall ist; A$^{n-}$ wenigstens ein n-wertiges Anion ist; x = $\sum$ny; und 0 < x ≤ 0,5, 0 ≤ m ≤ 10.

**31.** Pharmazeutische Formulierung gemäß Anspruch 30, wobei in der Formel (II) x ≥ 0,3 ist.

**32.** Pharmazeutische Formulierung gemäß Anspruch 30, wobei in der Formel (II) x < 0,3 ist.

**33.** Verfahren zum Herstellen einer pharmazeutischen Formulierung gemäß einem der Ansprüche 26 bis 32, wobei das Verfahren das Zusammenmischen der phosphatbindenden festen Mischmetallverbindung, des pharmazeutisch verträglichen Trägers und gegebenenfalls anderer Inhaltsstoffe umfasst.

**Revendications**

**1.** Substance pour utilisation en tant que médicament, comprenant un composé métallique mixte solide se liant au phosphate de formule (I) :

$$M^{II}_{1-a}M^{III}_aO_bA^{n-}_C.zH_2O \qquad (I)$$

où M$^{II}$ représente au moins un métal divalent ; M$^{III}$ représente Fe$^{3+}$ ; A$^{n-}$ représente au moins un anion n-valent ; a = moles de M$^{III}$/ (moles de M$^{II}$ + moles de M$^{III}$) ; 2+a = 2b+$\sum$cn et $\sum$cn < 0,9a, où le composé de formule (I) peut être obtenu par chauffage d'un produit de départ comprenant une structure en couche de type double hydroxyde à une température comprise entre 250 °C et 500 °C pendant 3 heures ou moins.

**2.** Substance pour utilisation en tant que médicament selon la revendication 1, où dans la formule (I), a est ≥ 0,3.

**3.** Substance pour utilisation en tant que médicament selon la revendication 1, où dans la formule (I), a est < 0,3.

**4.** Substance pour utilisation en tant que médicament selon la revendication 2, où dans la formule (I), 0,03a < $\sum$cn < 0,5a.

**5.** Substance pour utilisation en tant que médicament selon la revendication 3, où dans la formule (I), 0,03a < $\sum$cn < 0,7a.

**6.** Substance pour utilisation en tant que médicament selon l'une quelconque des revendications précédentes, où le composé métallique mixte solide comporte moins de 2 %, préférentiellement moins de 1,5 %, plus préférentiellement moins de 1 % en masse d'eau absorbée à la surface des cristallites.

**7.** Substance pour utilisation en tant médicament, comprenant un composé métallique mixte solide obtenu ou pouvant être obtenu par chauffage d'un produit de départ comprenant une structure en couche de type double hydroxyde à une température comprise entre 250 °C et 500 °C pendant 3 heures ou moins.

**8.** Substance pour utilisation en tant que médicament selon la revendication 7, où le produit de départ comprend un composé de formule (II) :

$$M^{II}_{1-x}M^{III}_x(OH)_2A^{n-}_y.mH_2O \qquad (II)$$

où M$^{II}$ représente au moins un métal divalent ; M$^{III}$ représente au moins un métal trivalent ; A$^{n-}$ représente au moins un anion n-valent ; x = Eyn, 0 < x ≤ 0,5, 0 < y ≤ 1 et 0 < m ≤ 10.

**9.** Substance pour utilisation en tant que médicament selon la revendication 8, où dans la formule (II), x est ≥ 0,3.

**10.** Substance pour utilisation en tant que médicament selon la revendication 8, où dans la formule (II), x est < 0,3.

**11.** Substance pour utilisation en tant que médicament selon l'une quelconque des revendications 7 à 10, où la substance présente une capacité de liaison au phosphate supérieure de 10 % à celle du composé de formule (II) à partir duquel elle peut être obtenue.

**12.** Utilisation dans la fabrication d'un médicament destiné à se lier au phosphate chez un animal le nécessitant d'une substance comprenant un composé métallique mixte solide se liant au phosphate de formule (I) :

$$M^{II}_{1-a}M^{III}_{a}O_{b}A^{n-}_{c} \cdot zH_2O \qquad \text{(I)}$$

où $M^{II}$ représente au moins un métal divalent ; $M^{III}$ représente $Fe^{3+}$ ; $A^{n-}$ représente au moins un anion n-valent ; a = moles de $M^{III}$/(moles de $M^{II}$ + moles de $M^{III}$) ; $2+a = 2b+\sum cn$ et $\sum cn < 0,9a$, où le composé de formule (I) peut être obtenu par chauffage d'un produit de départ comprenant une structure en couche de type double hydroxyde à une température comprise entre 250 °C et 500 °C pendant 3 heures ou moins.

13. Utilisation dans la fabrication d'un médicament destiné au traitement prophylactique ou thérapeutique de l'hyperphosphatémie d'une substance comprenant un composé métallique mixte solide se liant au phosphate de formule (I) :

$$M^{II}_{1-a}M^{III}_{a}O_{b}A^{n-}_{c} \cdot zH_2O \qquad \text{(I)}$$

où $M^{II}$ représente au moins un métal divalent ; $M^{III}$ représente $Fe^{3+}$ ; $A^{n-}$ représente au moins un anion n-valent ; a = moles de $M^{III}$/(moles de $M^{II}$ + moles de $M^{III}$) ; $2+a = 2b+Ecn$ et $\sum cn < 0,9a$, où le composé de formule (I) peut être obtenu par chauffage d'un produit de départ comprenant une structure en couche de type double hydroxyde à une température comprise entre 250 °C et 500 °C pendant 3 heures ou moins.

14. Utilisation selon la revendication 12 ou la revendication 13, où dans la formule (I), a est $\geq 0,3$ et préférentiellement $0,03a < \sum cn < 0,5a$.

15. Utilisation selon la revendication 12 ou la revendication 13, où dans la formule (I), a est $< 0,3$ et préférentiellement $0,03a < \sum cn < 0,7a$.

16. Utilisation dans la fabrication d'un médicament destiné à se lier au phosphate chez un animal le nécessitant d'une substance pouvant être obtenue par chauffage d'un produit de départ comprenant une structure en couche de type double hydroxyde à une température comprise entre 250 °C et 500 °C pendant trois heures ou moins.

17. Utilisation dans la fabrication d'un médicament destiné au traitement prophylactique ou thérapeutique de l'hyperphosphatémie d'une substance pouvant être obtenue par chauffage d'un produit de départ comprenant une structure en couche de type double hydroxyde à une température comprise entre 250 °C et 500 °C pendant trois heures ou moins.

18. Utilisation selon la revendication 16 ou la revendication 17, où le produit de départ comprend un composé de formule (II) :

$$M^{II}_{1-x}M^{III}_{x}(OH)_2 A^{n-}_{y} \cdot mH_2O \qquad \text{(II)}$$

où $M^{II}$ représente au moins un métal divalent ; $M^{III}$ représente au moins un métal trivalent ; $A^{n-}$ représente au moins un anion n-valent ; $x = \sum ny$ ; et $0 < x \leq 0,5$ ; $0 \leq m \leq 10$.

19. Utilisation selon la revendication 18, où dans la formule (II), x est $\geq 0,3$.

20. Utilisation selon la revendication 18, où dans la formule (II), x est $< 0,3$.

21. Procédé de préparation d'une substance pour utilisation en tant que médicament, le procédé comprenant le chauffage d'un produit de départ comprenant une structure en couche de type double hydroxyde à une température comprise entre 250 °C et 500 °C pendant trois heures ou moins.

22. Procédé selon la revendication 21, où le produit de départ comprend un composé de formule (II) :

$$M^{II}_{1-x}M^{III}_{x}(OH)_2 A^{n-}_{y} \cdot mH_2O \qquad \text{(II)}$$

où $M^{II}$ représente au moins un métal divalent ; $M^{III}$ représente au moins un métal trivalent ; $A^{n-}$ représente au moins un anion n-valent ; $x = \sum ny$ ; et $0 < x \leq 0,5$, $0 \leq m \leq 10$.

23. Procédé selon la revendication 22, où dans la formule (II), x est $\geq 0,3$.

**24.** Procédé selon la revendication 22, où dans la formule (II), x est < 0,3.

**25.** Procédé selon l'une quelconque des revendications 21 à 24, où le produit de départ est essentiellement exempt de particules de taille supérieure à 100 $\mu$m.

**26.** Formule pharmaceutique comprenant : un composé métallique mixte solide se liant au phosphate de formule (I) :

$$M^{II}_{1-a}M^{III}_{a}O_{b}A^{n-}_{c}.\ zH_2O \qquad (I)$$

où $M^{II}$ représente au moins un métal divalent ; $M^{III}$ représente $Fe^{3+}$ ; $A^{n-}$ représente au moins un anion n-valent ; a = moles de $M^{III}$/ (moles de $M^{II}$ + moles de $M^{III}$) ; $2+a = 2b+\sum cn$ et $\sum cn < 0,9a$ ; et un vecteur pharmaceutiquement acceptable où le composé de formule (I) peut être obtenu par chauffage d'un produit de départ comprenant une structure en couche de type double hydroxyde à une température comprise entre 250 °C et 500 °C pendant 3 heures ou moins.

**27.** Formule pharmaceutique selon la revendication 26, où dans la formule (I), a est $\geq$ 0,3 et préférentiellement 0,03a $< \sum cn < 0,5a$.

**28.** Formule pharmaceutique selon la revendication 26, où dans la formule (I), a est < 0,3 et préférentiellement 0,03a $< \sum cn < 0,7a$.

**29.** Formule pharmaceutique comprenant un composé métallique mixte solide pouvant être obtenu par chauffage d'un produit de départ comprenant une structure en couche de type double hydroxyde à une température comprise entre 250 °C et 500 °C pendant trois heures ou moins, et un vecteur pharmaceutiquement acceptable.

**30.** Formule pharmaceutique selon la revendication 29, où le produit de départ comprend un composé de formule (II) :

$$M^{II}_{1-x}M^{III}_{x}(OH)_{2}A^{n-}y.\ mH_2O \qquad (II)$$

où $M^{II}$ représente au moins un métal divalent ; $M^{III}$ représente au moins un métal trivalent ; $A^{n-}$ représente au moins un anion n-valent ; $x = \sum ny$ ; et $0 < x \leq 0,5$, $0 \leq m < 10$.

**31.** Formule pharmaceutique selon la revendication 30, où dans la formule (II), x est $\geq$ 0,3.

**32.** Formule pharmaceutique selon la revendication 30, où dans la formule (II), x est < 0,3.

**33.** Procédé de préparation d'une formule pharmaceutique selon l'une quelconque des revendications 26 à 32, le procédé comprenant le mélangeage du composé métallique mixte solide se liant au phosphate, du vecteur pharmaceutiquement acceptable et éventuellement d'autres composants.

**EP 1 850 859 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9915189 A
- JP 2004089760 A

**Non-patent literature cited in the description**

- **OOKUBO et al.** *Journal Pharmaceutical Sciences,* November 1992, vol. 81 (11), 1139-1140
- **TEZUKA, S.** *Bull. Chem. Soc. Jpn.,* 2004, vol. 77, 2101-7
- Layered Double Hydroxides: Present and Future. Nova Science, 2001